# EUROPEAN PATENT SPECIFICATION

(11) **EP 4 222 500 B1**
(45) Date of publication and mention of the grant of the patent: **12.11.2025**
(21) Application number: 21807318.7
(22) Date of filing: 30.09.2021
(51) Int. Cl.: G01N 33/68

(54) **METHODS OF DETECTING ISOASPARTIC ACID**
VERFAHREN ZUM NACHWEIS VON ISOASPARAGINSÄURE
PROCÉDÉS DE DÉTECTION D'ACIDE ISOASPARTIQUE

(30) Priority: 01.10.2020 US 202063086557 P; 02.07.2021 US 202163218186 P
(43) Date of publication of application: 09.08.2023
(73) Proprietor: Amgen Inc., Thousand Oaks, CA 91320-1799 (US)
(72) Inventor: HUI, John On-Ting, Thousand Oaks, CA 91320-1799 (US); CAMPUZANO, Iain David Grant, Thousand Oaks, CA 91320-1799 (US)
(74) Representative: Grünecker Patent- und Rechtsanwälte PartG mbB
(86) International application number: PCT/US2021/052783
(87) International publication number: WO 2022/072583

(56) References cited:
- CACIA J ET AL: "Isomerization of an aspartic acid residue in the complementarity-determining regions of a recombinant antibody to human IgE: Identification and effect on binding affinity", BIOCHEMISTRY (EASTON), AMERICAN CHEMICAL SOCIETY, UNITED STATES, vol. 35, no. 6, 13 February 1996 (1996-02-13), pages 1897 - 1903, XP002094430, ISSN: 0006-2960, DOI: 10.1021/BI951526C
- PRESTA ET AL: "Selection, design, and engineering of therapeutic antibodies", JOURNAL OF ALLERGY AND CLINICAL IMMUNOLOGY, ELSEVIER, AMSTERDAM, NL, vol. 116, no. 4, 1 October 2005 (2005-10-01), pages 731 - 736, XP005094459, ISSN: 0091-6749, DOI: 10.1016/J.JACI.2005.08.003
- KATHARINA DIEPOLD ET AL: "Simultaneous Assessment of Asp Isomerization and Asn Deamidation in Recombinant Antibodies by LC-MS following Incubation at Elevated Temperatures", PLOS ONE, vol. 7, no. 1, 17 January 2012 (2012-01-17), pages e30295, XP055332818, DOI: 10.1371/journal.pone.0030295
- HUI JOHN O ET AL: "A Robust MALDI-PSD Method for Site Specific Identification of Isomerized Aspartate Residue in Therapeutic Antibodies", 2ND ADVANCING MASS SPECTROMETRY FOR BIOPHYSICS AND STRUCTURAL BIOLOGY, 19 July 2019 (2019-07-19), University of Massachusetts Amherst, pages 1 - 93, XP055871328, Retrieved from the Internet <URL:https://advancingms.org/wp-content/uploads/2019/07/AMS_Conference_Program_Final_7_19_2019.pdf> [retrieved on 20211209]
- "Characterization of Protein Therapeutics using Mass Spectrometry", 1 July 2014, SPRINGER, article CHEN WEIBIN ET AL: "Applications of Ion Mobility Mass Spectrometry for Characterization of Protein Therapeutics", pages: 1 - 403, XP055871316
- DEGRAAN-WEBER NICK ET AL: "Distinguishing Aspartic and Isoaspartic Acids in Peptides by Several Mass Spectrometric Fragmentation Methods", JOURNAL OF THE AMERICAN SOCIETY FOR MASS SPECTROMETRY, ELSEVIER SCIENCE INC, US, vol. 27, no. 12, 9 September 2016 (2016-09-09), pages 2041 - 2053, XP036315862, ISSN: 1044-0305, [retrieved on 20160909], DOI: 10.1007/S13361-016-1487-9
- HUI JOHN O. ET AL: "Unequivocal Identification of Aspartic Acid and iso Aspartic Acid by MALDI-TOF/TOF: From Peptide Standards to a Therapeutic Antibody", JOURNAL OF THE AMERICAN SOCIETY FOR MASS SPECTROMETRY, vol. 32, no. 8, 4 August 2021 (2021-08-04), US, pages 1901 - 1909, XP055871194, ISSN: 1044-0305, Retrieved from the Internet <URL:https://pubs.acs.org/doi/pdf/10.1021/jasms.0c00370> [retrieved on 20211209], DOI: 10.1021/jasms.0c00370

## Description

### CROSS REFERENCE TO RELATED APPLICATION

This application claims priority to U.S. Provisional Patent Application No. 63/086,557, filed October 1, 2020 and U.S. Provisional Patent Application No. 63/218,186, filed July 2, 2021.

This application is being filed with a sequence listing in electronic format. The sequence listing provided as a file titled, "55797_Seqlisting.txt," created September 20, 2021, and is 7,091 bytes in size.

### BACKGROUND

The functions served by protein post-translational modifications (PTMs) such as arginine methylation¹, phosphorylation², ubiquitination³, glycosylation⁴⁻⁵ and O-linked β-N-acetylglucosamine modification⁶⁻⁷ in the biology of the cell have now been well established. Proteins can also undergo spontaneous chemical degradation, readily detectable after aging or under stressed conditions⁹. The current literature contains many studies on the characterization of such chemical modifications identified in both cellular proteins and biotherapeutics¹⁰⁻¹². Examples of such chemical modifications include methionine and tryptophan oxidation, asparagine deamidation, as well as aspartate isomerization¹³⁻¹⁶. Most stable modifications display a very specific mass change such as +15.994 Da for tryptophan (Trp) and methionine (Met) oxidation and +0.984 Da for asparagine (Asn) deamidation¹⁸. However, with aspartate isomerization, there is no change in mass after the modification.

During the development of biotherapeutics for human application, it is advantageous to identify chemical liabilities that negatively impact the activity and stability of the candidate molecules as early as possible. The proteins are typically subjected to a series of forced degradation studies to determine their stability, appropriate shelf life, suitable storage and transport conditions, a process known as "Molecular Assessment"³⁰⁻³¹. The molecules that are affected by the chemical modifications may be deselected or be subjected to another round of protein engineering to mitigate the adverse effect³². Most chemical modifications can be identified by the liquid chromatography-tandem mass spectrometry (LC-MS/MS) and data processing³³⁻³⁵. Identification of aspartate isomerization presents a more challenging problem. Because of the resultant structural impact after the modification, isoAsp formation in several therapeutic mAb has been shown to result in loss of activity³⁶⁻³³. Its occurrence during biotherapeutics manufacturing must therefore be carefully monitored and controlled³⁹.

Thus, there is a need in the art for efficient methods of detection of isoaspartic acid (isoAsp).
Diepold et al. ("Simultaneous Assessment of Asp Isomerization and Asn Deamidation in Recombinant Antibodies by LC-MS following Incubation at Elevated Temperatures", PLOS ONE, vol. 7(1), 2012) concerns methods for assessing asparagine deamidation and aspartate isomerization by quantitative UPLC-MS. Hui et al. ("A Robust MALDI-PSD Method for Site Specific Identification of Isomerized Aspartate Residue in Therapeutic Antibodies", 2ND Advancing Mass Spectrometry for Biophysics and Structural Biology Meeting, 21 July 2019) concerns a MALDI-PSD mass spectrometry systems for detecting isoAsp in peptides. Chen and Chakraborty ("Applications of Ion Mobility Mass Spectrometry for Characterization of Protein Therapeutics", In: "Characterization of Protein Therapeutics using Mass Spectrometry", 1 July 2014) concerns methods for characterization of proteins by separating peptides based on their geometry using ion mobility spectroscopy prior to mass spetrometry. Degraan-Weber et al. ("Distinguishing Aspartic and Isoaspartic Acids in Peptides by Several Mass Spectrometric Fragmentation Methods", Journal of the American Society for Mass Spectrometry, vol. 27(12): 2014-2053, 2016) provides methods for the MALDI-PDS-based detection of isoAsp-containing peptides.

PRESTA ET AL.: "Selection, design, and engineering of therapeutic antibodies", JOURNAL OF ALLERGY AND CLINICAL IMMUNOLOGY, ELSEVIER, AMSTERDAM, NL, vol. 116, no. 4, 1 October 2005, pages 731-736 discloses the selection, design and engineering of therapeutic antibodies (e.g. title and abstract). It identifies the problem of isoaspartate formation and proposes the engineering of the protein to remove the isoAsp by substitution. PRESTA ET AL. refers to CACIA J ET AL.: "Isomerization of an aspartic acid residue in the complementarity-determining regions of a recombinant antibody to human IgE: Identification and effect on binding affinity", BIOCHEMISTRY (EASTON), AMERICAN CHEMICAL SOCIETY, UNITED STATES, vol. 35, no. 6, 13 February 1996, pages 1897-1903. In this document isoAspartate in Fabs is identified by a method involving (a) digesting the protein with papain into peptides, performing HPLC, N-terminally sequencing the fragmented peptides and subjecting some N-terminal peptides to mass spectrometry.

### SUMMARY

The present invention is defined in the claims. Disclosed herein are methods of processing a protein. The method comprises: (a) digesting a protein into peptides, (b) subjecting the peptides to mass spectrometry (such as tandem-mass spectrometry (MS/MS)) to fragment a peptide bond N-terminal to an *iso*aspartic acid (*iso*Asp) and measure said fragmentation by the mass spectrometry; (c) comparing the fragmentation to a threshold, wherein the fragmentation exceeding the threshold is indicative of the presence of the *iso*Asp in the peptide being analysed and ultimately in the protein; and (d) (i) rejecting the protein comprising the *iso*Asp, wherein rejection comprises halting production of the protein or a purification process of the protein, or disposind of a supply of the protein, or (ii) engineering the protein to remove the *iso*Asp.

In various embodiments, the methods described herein further comprises reducing the charge (z) of the peptides subject to mass spectrometry prior to (b). In some embodiments, the peptides subject to mass spectrometry in (b) are singly-charged. In any of the described methods, the fragmentation is measured as a b-series peak, a y-series peak, or both.

The disclosed methods may be carried out with any type of mass spectrometry methods. For example, the mass spectrometry for use in the methods described herein include matrix-assisted laser desorption/ionization time-of-flight/time-of-flight (MALDI-TOF/TOF) or liquid chromatography-mass spectrometry/mass-spectrometry (LC-MS/MS). In related embodiments, the LC-MS/MS comprises electrospray ionization.

In any of the disclosed methods, digesting a protein into peptides may be carried out after thermal stressing of the protein followed by enzymatic digestion with a proteolytic enzyme. For example, the proteolytic enzyme comprises at least one of chymotrypsin, trypsin, pepsin, papain, or elastase.

In various embodiments, the isoAsp is not adjacent to an asparagine in the amino acid sequence of the protein and/or is not adjacent to a threonine in the amino acid sequence of the protein.

In various embodiments, the *isoAsp* is adjacent to an adjacent aspartate residue in the amino acid sequence of the protein. In related embodiments, the adjacent aspartate is *isoAsp* or L-Asp.

In the disclosed methods, a threshold level of fragmentation of the peptide is used as a standard for determining whether *iso*Asp is present in the protein. For example, the threshold may be the level of fragmentation of the peptide bond of a control peptide, such as a synthetic peptide. In various embodiments, the fragmentation of the peptide exceeds the threshold when a b-peak and/or a y-peak of the fragmented peptide bond N-terminal to *iso*Asp is present.

In various embodiments, the methods described herein further comprise subjecting a control peptide to mass spectrometry, thereby obtaining the fragmentation of the peptide bond of the control peptide. In various embodiments, the control peptide is not subjected to thermal stressing. In related embodiments, the control peptide is a synthetic peptide. In various embodiments, the synthetic peptide may be used for comparison to one or more protein sample(s). In various embodiments, the threshold is the level of fragmentation of a peptide bond N-terminal to an L-Asp in a control peptide. In various embodiments, the fragmentation of the peptide bond of the control peptide is provided as an electronically-stored value or is provided from a reference peptide subjected to the same mass spectrometry methods as the protein sample being tested.

In any of the disclosed methods, the protein comprises or consists of an antibody, antibody protein product, bispecific T-cell engager (BiTE^{®}) molecule, antibody fragment, antibody fusion peptide or antigen-binding fragment thereof, peptide, growth factor, or cytokine. In related embodiments, the antibody is a polyclonal or monoclonal antibody. As used herein, the term "antibody protein product" refers to any one of several antibody alternatives which in various instances is based on the architecture of an antibody but is not found in nature. In some aspects, the antibody protein product has a molecular-weight within the range of at least about 12 kDa - 1 MDa, for example at least about 12 kDa - 750 KDa, at least about 12 kDa - 250 kDa, or at least about 12 kDa - 150 kDa. In certain aspects, the antibody protein product has a valency (n) range from monomeric (n = 1), to dimeric (n = 2), to trimeric (n = 3), to tetrameric (n = 4), if not higher order valency. Antibody protein products in some aspects are those based on the full antibody structure and/or those that mimic antibody fragments which retain full antigen-binding capacity, e.g., scFvs, Fabs and VHH/VH (discussed below). The smallest antigen binding antibody fragment that retains its complete antigen binding site is the Fv fragment, which consists entirely of variable (V) regions. A soluble, flexible amino acid peptide linker is used to connect the V regions to a scFv (single chain fragment variable) fragment for stabilization of the molecule, or the constant (C) domains are added to the V regions to generate a Fab fragment [fragment, antigen-binding]. Both scFv and Fab fragments can be easily produced in host cells, e.g., prokaryotic host cells. Other antibody protein products include disulfide-bond stabilized scFv (ds-scFv), single chain Fab (scFab), as well as di- and multimeric antibody formats like dia-, tria- and tetra-bodies, or minibodies (miniAbs) that comprise different formats consisting of scFvs linked to oligomerization domains. The smallest fragments are VHH/VH of camelid heavy chain Abs as well as single domain Abs (sdAb). The building block that is most frequently used to create novel antibody formats is the single-chain variable (V)-domain antibody fragment (scFv), which comprises V domains from the heavy and light chain (VH and VL domain) linked by a peptide linker of ~15 amino acid residues. A peptibody or peptide-Fc fusion is yet another antibody protein product. The structure of a peptibody consists of a biologically active peptide grafted onto an Fc domain. Peptibodies are well-described in the art. See, e.g., Shimamoto et al., mAbs 4(5): 586-591 (2012). Other antibody protein products include a single chain antibody (SCA); a diabody; a triabody; a tetrabody; bispecific or trispecific antibodies, and the like. Bispecific antibodies can be divided into five major classes: BslgG, appended IgG, BsAb fragments, bispecific fusion proteins and BsAb conjugates. See, e.g., Spiess et al., Molecular Immunology 67(2) Part A: 97-106 (2015). In exemplary aspects, the antibody protein product comprises or consists of a bispecific T cell engager (BiTE^{®}) molecule, which is an artificial bispecific monoclonal antibody. BiTE^{®} molecules are fusion proteins comprising two scFvs of different antibodies. One binds to CD3 and the other binds to a target antigen. BiTE^{®} molecules are known in the art. See, e.g., Huehls et al., Immuno Cell Biol 93(3): 290-296 (2015); Rossi et al., MAbs 6(2): 381-91 (2014); Ross et al., PLoS One 12(8): e0183390.

In various embodiments, the methods described herein further comprise determining the position of the i*soA*sp in the protein. In various embodiments, the isoAsp is detected during a protein discovery process or during a protein production process. In various embodiments, absence of the i*so*Asp indicates suitability of the protein for further processing.

In various embodiments, the method further comprises rejecting the protein when the protein comprises i*so*Asp. "Rejecting" the protein includes, but is not limited to, halting or revising the protein production or purification process or disposing of the protein supply. If the protein production process is halted, additional processing may be carried out to further assess protein stability, molecular assessment, and/or quality control. In various embodiments, rejecting the protein comprises discarding or disposing of a quantity of product comprising the protein, and/or rejecting a clone that produces the protein.

In various embodiments wherein the method determines the protein comprises isoAsp, the method further comprises engineering the protein to remove the site that is susceptible to *iso*Asp formation. For example, the engineering comprises inducing a point mutation at the position of the isoAsp in the protein.

In various embodiments, the presence of isoAsp results in structural changes in the peptide backbone compared to a reference protein comprising an L-Asp at the same position.

In various embodiments, the presence of *iso*Asp inhibits potency of the protein. In various embodiments, the presence of isoAsp increases the immunogenicity of the protein compared to a reference protein comprising an L-Asp at the same position. In addition, the disclosure provides for methods of evaluating the potency and/or immunogenicity of a protein by determining whether the protein comprises *iso*Asp.

In any of the described methods, at least one of the peptides comprises a positive charge at the N terminus of the peptide.

In various embodiments and prior to subjecting the peptide to mass spectrometry analysis, the method further comprises introducing a positive charge to the N terminus of the peptide. In various embodiments, the positive charge is introduced by incubating the peptide with an N-terminal charge-derivatizing reagent. In certain embodiments, the N-terminal charge-derivatizing reagent is (N-succinimidyloxycarbonylmethyl) tris (2,4,6 trimethoxyphenyl) phosphonium bromide (TMPP) or a derivative thereof. In certain embodiments, the N-terminal charge-derivatizing reagent is tris[2,4,6-trimethoxyphenyl]phosphonium acetyl (TMPP-Ac) or a derivative thereof. In various embodiments, the *iso*Asp is within 5 amino acid residues of a C-terminus of at least one of the peptides, for example within 4, 3, 2, or 1 amino acid residues of the C-terminus.

In another aspect, disclosed herein, but not part of the invention as defined in the claims, is a mass spectrometry system configured for performing the methods described herein. In various embodiments, the fragmentation of a peptide bond N-terminal to the isoAsp is measured using the mass spectrometry system disclosed herein. In related embodiments, the fragmentation exceeding the threshold is indicative of the presence of isoAsp in a protein. In addition, the disclosure provides for methods of determining the presence of isoAsp in a protein using a mass spectrometry system of the disclosure.

In various embodiments, the mass spectrometry performed is matrix-assisted laser desorption/ionization time-of-flight/time-of-flight (MALDI-TOF/TOF). In various embodiments, the mass spectrometry performed is liquid chromatography-mass spectrometry/mass-spectrometry (LC-MS/MS), such as electrospray ionization LC-MS/MS.

The use of the singular includes the plural unless specifically stated otherwise. The use of "or" means "and/or" unless stated otherwise. The use of the term "including", as well as other forms, such as "includes" and "included," is not limiting.

"About" or "approximately" means, when modifying a quantity (e.g., "about" 3:1 ratio), that variation around the modified quantity can occur. These variations can occur by a variety of means, such as typical measuring and handling procedures, inadvertent errors, ingredient purity, and the like.

"Comprising" and "comprises" are intended to mean that methods include the listed elements but do not exclude other unlisted elements. The terms "consisting essentially of" and "consists essentially of," when used in the disclosed methods include the listed elements, exclude unlisted elements that alter the basic nature of the method, but do not exclude other unlisted elements. Embodiments defined by each of these transition terms are within the scope of this disclosure.

### BRIEF DESCRIPTION OF THE DRAWINGS

Figure 1 is a schematic displaying the isomerization pathway of both amino acid residues, aspartic acid and asparagine, via the succinimide intermediate, to form isoaspartic acid. The chirality and equilibrium of each stage was not considered.

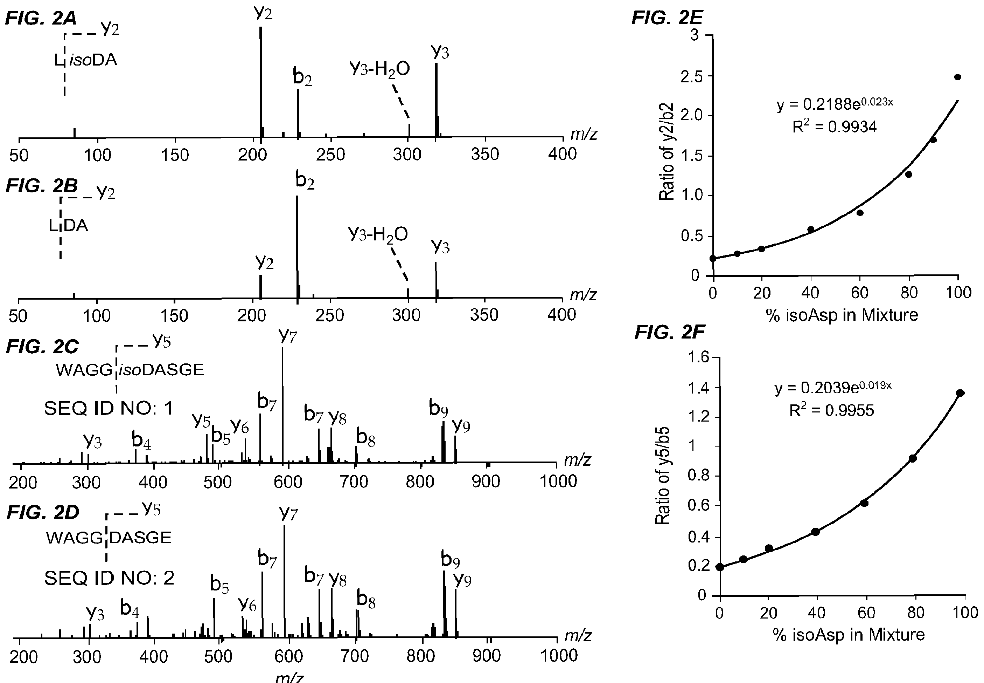

Figures 2A-2F are mass spectra and graphs of matrix-assisted laser desorption/ionization time-of-flight/time-of-flight (MALDI-TOF/TOF) spectrometry performed on standard peptides; Fig. 2A) L*i*s*o*DA; Fig. 2B) LDA; Fig. 2C) A plot of the ratio y₂/b₂ against %-isoAsp peptide in a defined mixture of the two tripeptides. Each point represents the average of triplicate measurements. Fig. 2D) β-Asp DSIP (WAGGisoDASGE; SEQ ID NO: 1; Fig. 2E) DSIP (WAGGDASGE; SEQ ID NO: 2; Fig. 2F) A plot of the ratio y₅/bₛ against %-isoAsp peptide in a defined mixture of the two nonapeptides. Each point is again the average of triplicate measurements.
Figures 3A-3F are mass spectra showing the identification of peptides containing isoAsp residue from thermal stressed therapeutic mAb by MALDI-TOF/TOF. Each peptide was collected and analyzed by MALDI-TOF/TOF.
Figures 4A-4B are a series of mass spectra of ESI infusion of the peptides WAGGDASGE (Fig. 4A; SEQ ID NO: 2) and WAGGisoDASGE (Fig. 4B; SEQ ID NO: 1) into the Orbitrap Velos Pro showed the unmodified peptide displayed only the singly charged ion.
Figure 5 is a series of mass spectra of MALDI-TOF/TOF fragmentation of the control (top) and the modified (bottom) peptide. The enhancement in the b₉ and the y₈ ions were observed in the isoD peptide. Using the ratio between these two ions in each spectrum, Rᵢₛₒₘₑᵣ was determined to be 1.8.
Figure 6 is a series of mass spectra of MALDI TOF/TOF analysis of a synthetic control (top panel) and modified (bottom panel) peptide. Enhancement of the y₉ ion was observed in the isoD peptide. By using the y₉ and y₁₀ pair for the calculation, Rᵢₛₒₘₑᵣ was determined to be 3.1.
Figure 7 is a series of mass spectra showing the enhancement of the y₄ ion was readily observed in the modified peptide, indicative that the peptide bond N-terminal to the isoD residue was more easily fragmented in MALDI TOF/TOF. By using the y₄ and y₃ pair, Rᵢₛₒₘₑᵣ was calculated to be 3.7.
Figure 8 is a series of mass spectra of MALDI TOF/TOF fragmentation of the pentapeptide ALDGE (top) (SEQ ID NO: 8) and ALisoDGE (bottom) (SEQ ID NO: 9). By using the y₈ and b₄ pair, Rᵢₛₒₘₑᵣ was determined to be 2.3. It is interesting to note that the b₃ fragment ion in the modified peptide was very much reduced in comparison to the control peptide.
Figure 9 is a series of mass spectra of MALDI TOF/TOF fragmentation of ALDEK (top) (SEQ ID NO: 10) and ALisoDEK (bottom) (SEQ ID NO: 11) shows an enhancement in the y₃ ion in the modified peptide. By using y₃ and b₄ in the calculation, Rᵢₛₒₘₑᵣ was determined to be 1.5. Again, the b₃ fragment was essentially not detected in the *iso*D peptide.
Figure 10 is a series of mass spectra of MALDI-TOF/TOF fragmentation of ALDGK (top) (SEQ ID NO: 12) and ALisoDGK (bottom) (SEQ ID NO: 13). By using the y₂ and y₃ pair, Rᵢₛₒₘₑᵣ was determined to be 2.1.
Figure 11 is a series of mass spectra of MALDI-TOF/TOF fragmentation of GLDLLK (top) (SEQ ID NO: 14) and GLisoDLLK (bottom) (SEQ ID NO: 15) showed an enhancement in the y₄ fragment observed with the modified peptide. By using the y₄ and y₂ fragments of each peptide, Rᵢₛₒₘₑᵣ was determined to be 2.8.
Figure 12 is a series of mass spectra of MALDI-TOF/TOF fragmentation of GDLLLK (top) (SEQ ID NO: 16) and GisoDLLLK (bottom) (SEQ ID NO: 17). While the fragment N-terminal to the modified residue (y₅) is minimal, it is significantly larger than that detected in the control peptide. Using y₅ and b₄ in the calculation, Rᵢₛₒₘₑᵣ was determined to be 5.8.
Figures 13A-13B are a series of mass spectra of liquid chromatography-tandem mass spectrometry (LC-MS/MS) analysis of the singly charged species (M+H+ ; m/z 1204.48) of the chymotryptic peptide encompassing Asp102 showed the modified peptide gave a very prominent b₆ + H₂O fragment. The data is very similar to what was observed using MALDI-TOF/TOF.

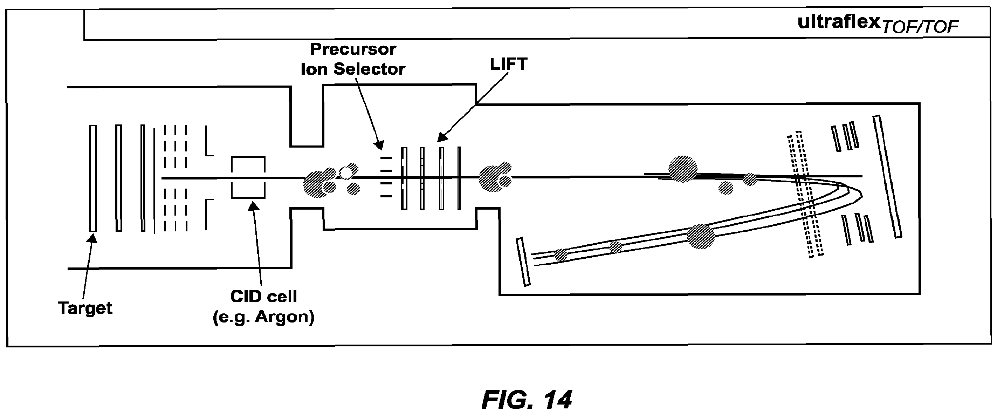

Figure 14 is an instrument schematic of the MALDI-TOF/TOF instrument displaying peptide fragmentation and the LIFT technology.

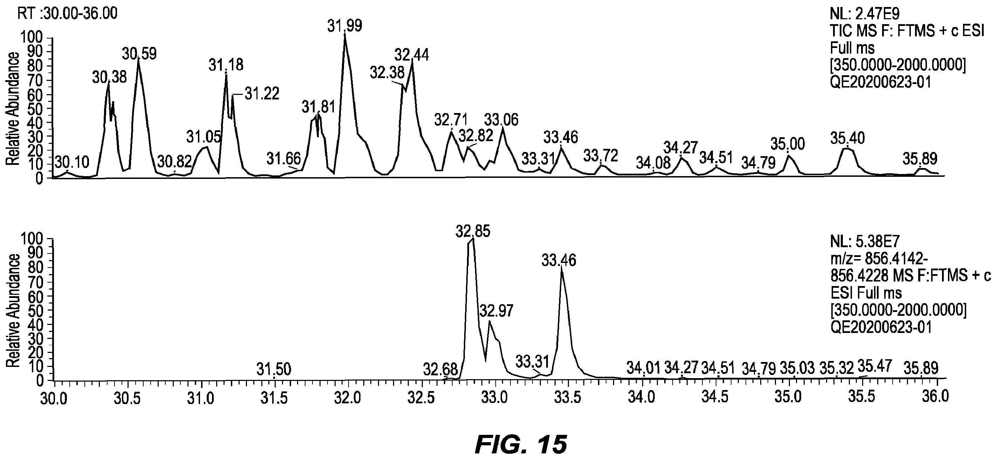

Figure 15 is a series of chromatograms showing Red/lAM of a heat-stressed antibody protein product Tryptic peptide (XIC of the peptide of interest)
Figure 16 is a chromatogram showing a heat-stressed antibody protein product Tryp. Peaks P1, P2 and P4 have the same m/z. P3 is the cyclic imide derivative XDDHXXXXXXXXXXXXXXXXXXXXXXX. The suspected isoAsp is DD and DH or DDH, with isoAsp residue underlined.

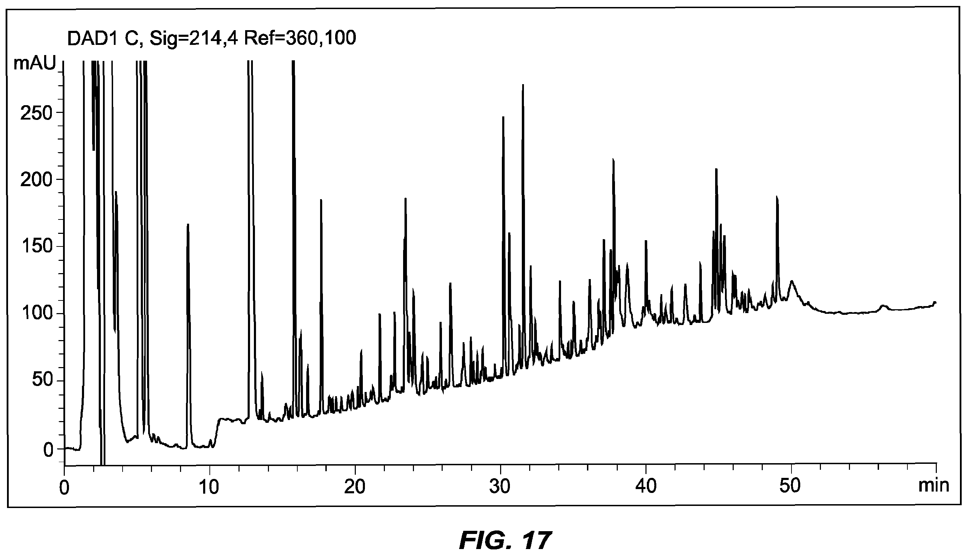

Figure 17 is an LC chromatographic trypsin map of a heat-stressed antibody protein product following thermal stressing.

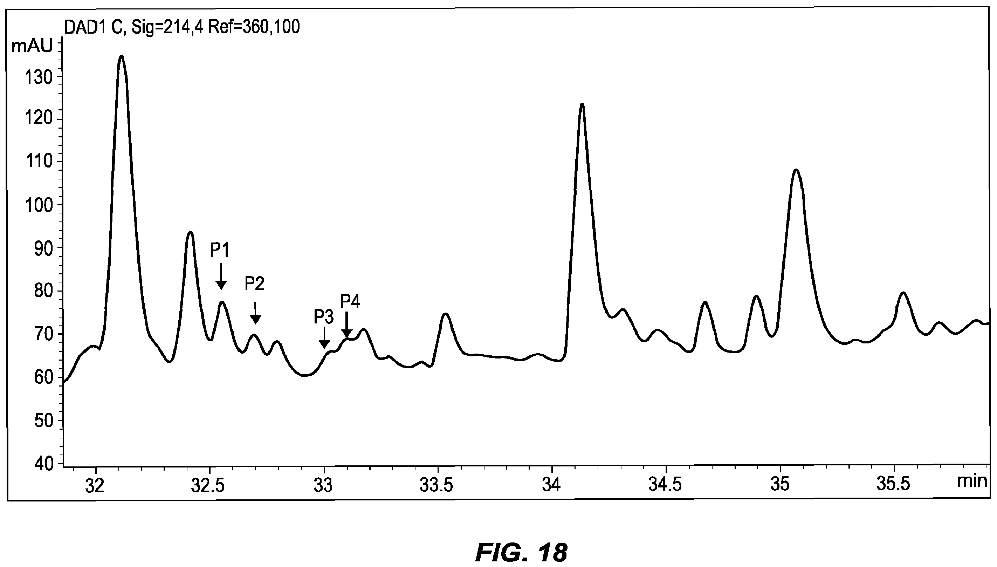

Figure 18 is a chromatogram showing the location of the 4 peptides of interest.
Figure 19 shows a spectrum following MALDI fragmentation of the peptide of interest derived from the control (unstressed) protein (top panel) and the heat stressed material P1 (bottom panel). In both cases, the ratio of the intensity of y₂₅ to y₂₃ is approximately 1:1, indicating P1 is the remaining unmodified peptide.
Figure 20 shows a spectrum following MALDI fragmentation of the peptide derived from heat stressed material P2 (top panel) and P4 (bottom panel). With P2, y₂₅ to y₂₃ was determined to be 1.7 to 1, indicating the modification is at isoDH. With P4, the readily detectable y₂₆ demonstrates the modification is at isoDD.

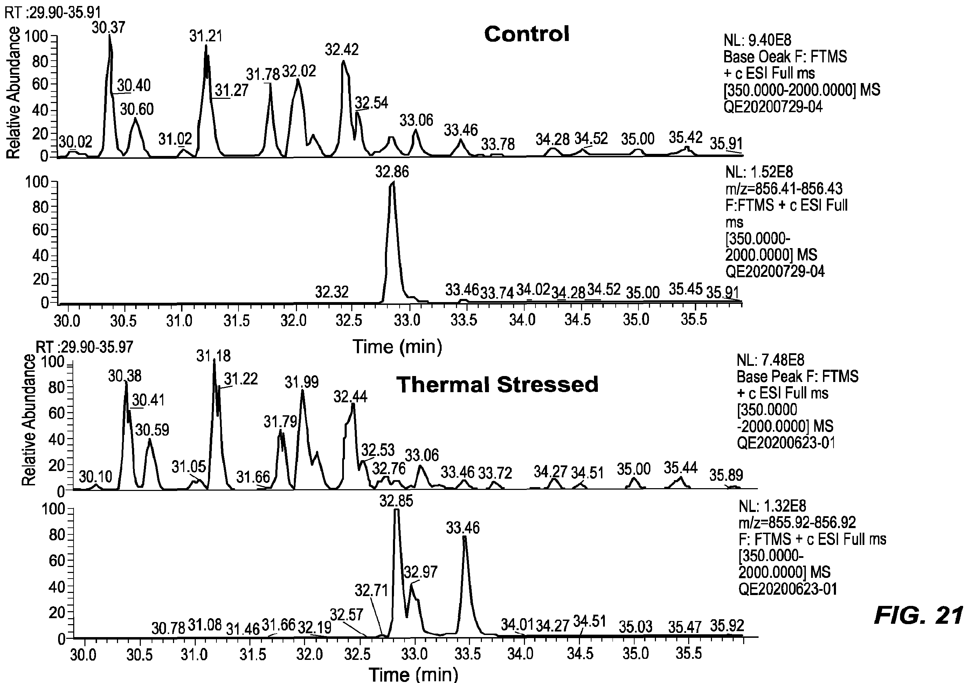

Figure 21 show mass spectra of control versus thermally stressed antibody protein product sample.
Figures 22A-22B: Figure 22A shows MALDI-TOF/TOF fragmentation of GFYPSDIAVEWESNGQPEDNYK (SEQ ID NO: 24) (top panel) & GFYPSDIAVEWESNGQPEisoDNYK (SEQ ID NO: 25) (bottom panel). Figure 22B shows MALDI-TOF/TOF fragmentation of the peptide GFYPSDIAVEWESNGQPENDYK (SEQ ID NO: 26) (top panel) & GFYPSDIAVEWESNGQPENisoDYK (SEQ ID NO: 27) (bottom panel).
Figures 23A-23B: Figure 23A shows MALDI-TOF/TOF fragmentation of TMPP modified GFYPSDIAVEWESNGQPEDNYK (SEQ ID NO: 24) (top panel) & TMPP modified GFYPSDIAVEWESNGQPEisoDNYK (SEQ ID NO: 25) (bottom panel). Figure 23B shows MALDI-TOF/TOF fragmentation of TMPP modified GFYPSDIAVEWESNGQPENDYK (SEQ ID NO: 26) (top panel) & TMPP modified GFYPSDIAVEWESNGQPENisoDYK (SEQ ID NO: 27) (bottom panel).
Figure 24 shows an exemplary structure of (N-Succinimidyloxycarbonylmethyl)tris(2,4,6-trimethoxyphenyl)phosphonium bromide (TMPP).

### DETAILED DESCRIPTION

Disclosed herein are methods of processing a protein. In exemplary aspects, the protein is a large peptide/ polypeptide (> 50 amino acids), antibody, antibody protein product, bispecific T cell engager (BiTE^{®}) molecule, antibody fragment, antibody fusion peptide or antigen-binding fragments thereof. In exemplary embodiments, the method comprises using mass spectrometry to detect isoAsp in a protein. The disclosed methods are advantageous, because, in contrast to conventional detection of *iso*Asp, neither specialized instrumentation nor ion chemistry is required. The present disclosure also provides mass spectrometry systems configured for performing the methods disclosed herein. In some embodiments, the protein has at least two consecutive aspartate residues in its amino acid sequence.

In exemplary embodiments, the method comprises: (a) digesting a protein into peptides, (b) subjecting the peptides to mass spectrometry (such as tandem-mass spectrometry) to fragment a peptide bond N-terminal to an *iso*Asp and measure said fragmentation by the mass spectrometry; (c) comparing the fragmentation to a threshold, wherein the fragmentation exceeding the threshold is indicative of the presence of the *iso*Asp in the protein; and (d) rejecting the protein comprising the *iso*Asp wherein rejection comprises halting production of the protein or a purification process of the protein, or disposing of a supply of the protein, or engineering the protein to remove the *iso*Asp. As used herein, *iso*Asp, unless stated otherwise, refers to an *iso*Asp residue in the context of a protein or peptide. Further description of the method is provided below. It is contemplated that small amounts of *iso*Asp may be tolerated for some applications. Accordingly, (d) comprises rejecting the protein if the *iso*Asp is at or above a specified level. In some embodiments, the method further comprises introducing a positive charge to the N terminus of the peptides using an N-terminal charge-derivatizing reagent, such as TMPP, prior to subjecting the peptide to mass spectrometry.

The experiments provided herein demonstrate that the peptide bond N-terminal to an *iso*Asp residue within the amino acid sequence of a peptide or protein was more susceptible to MALDI-TOF/TOF fragmentation. This finding was employed to distinguish an *iso*Asp containing peptide from its unmodified counterpart. In various embodiments, the method comprises measuring, by mass spectrometry, the fragmentation of a peptide bond N-terminal to an *iso*Asp residue. Mass spectrometry is used to measure the mass/charge of ions. In some embodiments, the charge is associated with the C-terminal region of the peptide and is known as the "y-series peak" or "y-peak". In some embodiments, the charge is associated with the N-terminal region of the peptide and is known as the "b-series peak" or "b-peak". In some embodiments, the fragmentation is measured as a b-series peak, a y-series peak, or both.

In various embodiments, to determine the presence of *iso*Asp in a protein sample, the fragmentation of a protein sample is compared to a threshold, where the fragmentation exceeding the threshold is indicative of the presence of the *iso*Asp in the protein. The threshold may be determined by measuring fragmentation of a control peptide or the threshold may be a reference value that is known to correspond with fragmentation of unmodified proteins, such as protein that does not comprise *iso*Asp. In some embodiments, the threshold may be based on presence of a b-peak and/or a y-peak of the fragmented peptide bond N-terminal to *iso*Asp. In related embodiments, the fragmentation exceeds the threshold when a b-peak and/or a y-peak of the fragmented peptide bond N-terminal to *iso*Asp is present in the protein sample.

In some embodiments, the fragmentation of a protein sample is compared to the fragmentation to that of a control peptide. The control peptide is also referred to as a "reference peptide." The control or reference peptide may be a peptide or protein which does not contain an *iso*Asp residue or has been engineered to remove *iso*Asp and/or contain an L-Asp residue. In related embodiments, the threshold may be based on the level of fragmentation of a peptide bond N-terminal to an L-Asp in a control peptide. The threshold refers to a level of fragmentation that is indicative of the presence of at least one isoAsp in a protein or peptide.

In some embodiments, the control peptide is optionally subjected to the same method (including (a), (b) and/or (c)) as the protein sample. In some embodiments, the fragmentation of the peptide bond of the control peptide is provided as an electronically-stored value or a reference value. An exemplary reference value is based on mass spec data from previously processed proteins that is stored electronically. In some instances, processing software generate electronic rules on *iso*Asp fragmentation and intensity of fragment ions N-terminal to the *iso*Asp when compared to a control peptide. The control peptide may be a synthetic peptide particularly designed based on the protein being processed. Alternatively, the control peptide may be a fragment of a protein that does not comprise isoAsp and preferably does not comprise any modified or altered amino acids. In particular, the control protein comprises an L-Asp at the same position as the protein in the sample.

It was also demonstrated that higher energy collisional dissociation (HCD) by electrospray LC-MS/MS of only the singly charged ion of an isoAsp containing peptide produced similar tandem mass spectrometry (MS/MS) fragmentation to that generated by MALDI-TOF/TOF.

### Digestion

In various embodiments, the disclosed methods comprise digesting a protein sample to be tested, into peptides. The digestion may be carried out using any known methods that would digest a protein into peptides. For example, digestion of the sample protein may be carried out using enzymatic digestion using a proteolytic enzyme after thermal stressing has been performed.

Thermal degradation refers to deterioration of polymeric molecules as a result of exposing proteins and/or peptides to increased temperatures, and in some instances excessively high temperatures. In various embodiments, thermal degradation is carried out at 38-400°C. In exemplary embodiments, thermal degradation is carried out at 40°C for 2-4 weeks, 3-6 months at 40°C, 1-2 months at 25°C, 1 month at 25°C, 3-6 months at 4°C or 6 months at 4°C. In various embodiments, thermal degradation is carried out at 4, 5, 6, 7, 8, 9, 10, 15, 20, 25, 30, 35, 40, 41, 42, 43, 44, 45, 46, 47, 48, 49, 50, 55, 60, or 65°C. In various embodiments, thermal degradation is carried out for 1 hour, 2 hours, 4 hours, 8 hours, 12 hours, 16 hours, 24 hours or 1 day, 2 days, 3, days, 4 days, 5 days, 6 days, 7 days or 1 week, 8 days, 9 days, 10 days, 11 days, 12 days, 13 days, 14 days or at least 2 weeks; at least 3 weeks, at least 4 weeks or at least 1 months, at least 5 weeks, at least 6 weeks, at least 8 weeks or at least 2 months, at least 3 months, at least 4 months, at least 5 months, or at least 6 months. In various embodiments, the thermal degradation is carried out between pH 5-8. In exemplary embodiments, thermal degradation is carried out at pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or pH 8.0. In various embodiments, the thermal degradation is performed accordingly to the International Council for Harmonisation of Technical Requirements for Pharmaceuticals for Human Use (ICH) guidelines. In some embodiments, thermal degradation comprises incubation at 40°C for 2-4 weeks (such as 2 weeks) at a pH in a range of pH 5 to pH 8.

Enzymatic digestion of proteins involves cleaving the peptide bond with an enzyme to form peptides. The digestion may be carried out with enzymes having varying degrees of specificity. For example, enzymatic digestion may be carried out with one or more proteolytic enzymes. Proteolytic enzymes include, but are not limited to, chymotrypsin, trypsin, pepsin, papain, or elastase. In some embodiments, the protein sample may be reduced and alkylated prior to digestion with a proteolytic enzyme and subsequently analyzed by mass spectrometry. The protein may be reduced with redox agents such as dithiothreitol (DTT), ß-mercaptoethanol and TCEP (Tris (2-carboxyethyl) phosphine). The protein may be alkylated with a sulfhydryl reagent such as iodoacetamide (IAM), iodoacetic acid (IAA) or another electrophile to prevent reformation of disulfide linkages. In various embodiments, both the protein sample and control sample (control peptide) are digested with a proteolytic enzyme. In various embodiments, the protein sample is thermally degraded and subsequently analyzed by mass spectrometry.

### Chemical modification of isoaspartate containing peptides with a N-terminal charge-derivatizing reagent

It is observed herein that introducing a positive charge to the N terminus of the peptide(s) prior to mass spectrometry can enhance the intensity of the peak that identifies fragmentation N-terminal of *iso*Asp, such as the bₙ₋₁+H₂O peak (where n is the residue number of the isoAsp in the peptide) compared to a reference peptide in which the positive charge is not introduced to the N-terminus (See Example 7). In various embodiments, the methods disclosed herein may further comprise N-terminal modification of peptides. In various embodiments, the method further comprises introducing a positive charge to the N terminus of the peptides, prior to mass spectrometry analysis. In various embodiments, the positive charge is introduced by incubating the peptides with an N-terminal charge-derivatizing reagent. An "N-terminal charge-derivatizing reagent" refers to reagents which have the ability add a positive charge to the N terminus of one or more peptides or proteins (i.e. agent which introduce a positively charged group, such as quaternary or tertiary ammonium and quaternary phosphonium groups at the N-terminus of a peptide or protein). Exemplary N-terminal charge-derivatizing reagents which may be used with methods disclosed herein include, but are not limited to, (N-succinimidyloxycarbonylmethyl) tris (2,4,6 trimethoxyphenyl) phosphonium bromide (TMPP), tris[2,4,6-trimethoxyphenyl]phosphonium acetyl (TMPP-Ac), 4-amidinobenzoic acid, nicotinic acid, 2-(6-amidinonaphthalen-2-yloxy)acetic acid, 4-(guanidinomethyl)benzoic acid, or derivatives thereof.

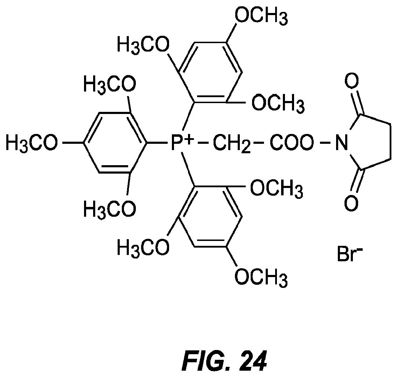

An exemplary structure of TMPP is shown in Figure 24. Without being limited by theory, it is contemplated that TMPP retains a positive charge on its phosphorus atom, permitting the positive charge to be directed to the N terminus of the peptide when the peptide is tagged with TMPP. The disclosed method can be carried out with reagents comprising the disclosed structure of TMPP or derivatives thereof. The disclosed method can also be carried out with regents that share a similar structure that adds a positive charge to the N-terminal of the peptide or protein.

In various embodiments, the introduction of a positive charge to the N terminus of one or more peptides, for example with a N-terminal charge-derivatizing reagent such as TMPP, may improve the detection of isoaspartate containing peptides by mass spectrometry. In various embodiments, an N-terminal charge-derivatizing reagent may be used when the isoAsp is within 5 amino acid residues of a C-terminus of at least one of the peptides. For example, the isoAsp may be within 4, 3, 2, or 1 amino acid residues of the C-terminus.

In certain embodiments the mass spectrometry method is MALDI-TOF/TOF. In exemplary embodiments, N-terminal modification of the peptide using a N-terminal charge-derivatizing reagent such as TMPP, followed by MALDI-TOF/TOF fragmentation, results in the detection of a more intense bₙ₋₁+H₂O product ion in the modified peptide when compared to the aspartate counterpart (n denotes the residue number of the isoaspartate in the peptide).

### Mass spectrometry

In exemplary aspects, the method comprises using mass spectrometry to detect isoAsp in a sample. Mass spectrometry is used to measure the mass/charge of ions. In various embodiments, the mass spectrometry comprises or consists of matrix-assisted laser desorption/ionization time-of-flight/time-of-flight (MALDI-TOF/TOF). In various embodiments, the mass spectrometry comprises or consists of liquid chromatography-mass spectrometry/mass-spectrometry (LC-MS/MS), also referred to as liquid chromatography-tandem mass spectrometry.

Disclosed herein is the use of mass spectrometry to detect and determine the position of *iso*Asp residues in peptides. The experiments described herein demonstrate that the peptide bond N-terminal to the modified *iso*Asp residue was more susceptible to MALDI-TOF/TOF fragmentation. In the present disclosure, the two terms MALDI-TOF/TOF and MALDI-LIFT-TOF/TOF are used interchangeably. In exemplary embodiments, MALDI-TOF/TOF mass spectrometry is used to characterize and determine the position of *iso*Asp residues in synthetic peptides and proteolytically generated peptides from a protein sample. In alternative embodiments, LC-MS/MS mass spectrometry may be used. Without being limited by theory, it is contemplated that MALDI-TOF can be used in methods described herein with charge reduction. For methods comprising LC-MS/MS, the mass spectrometry may be preceded by charge reduction.

In various embodiments, the methods described herein further comprise charge reduction of the peptide sample prior to (b). Charge reduction (Smith. J Am Soc Mass Spectrom 2008, 19(5), 629-31) is used to reduce peptide charge states to z = 1+ for detection of isoAsp in a protein sample by mass spectrometry. In some embodiments, the peptides subject to mass spectrometry in (b) are singly-charged. The term "singly-charged" refers to peptide with +1 or -1 charge state. In some embodiments, the fragmentation is measured as a b-series peak, a y-series peak, or both.

In various embodiments, "charge reduction" as used herein, refers to decreasing the magnitude of the charge of the peptide, so as to decrease the number of charged species in the peptide, which may make the charge state of the peptide neutral (or closer to neutral). In some embodiments, the charge of peptide is reduced by making it smaller (e.g. by enzymatic digestion). In some embodiments, the charge of peptide is reduced by a favorable addition (e.g. the use of a basic chemical). In exemplary embodiments, charge reduction can be performed in the following ways. Firstly, the length of the peptide is reduced by using an enzyme other than trypsin, such as chymotrypsin. The resulting peptide would be smaller and therefore possess less charge. In this case, the charge state would by z=1+, therefore now amenable to LC-MS/MS HCD fragmentation. Secondly, the use of a basic chemical such as 1,8 diazabicyclo[5.4.0]-undec-7-ene (DBU) for example, to induced gas-phase base mediated charge stripping, therefore reducing the charge of the peptide from z=3+ to z=1+, for example, allowing for LC-MS/MS HCD fragmentation of the z=1+ ion.

The present disclosure also provides mass spectrometry systems configured for performing the methods disclosed herein. In various embodiments, the fragmentation of a peptide bond N-terminal to the isoAsp is measured using the mass spectrometry system disclosed herein. In related embodiments, the fragmentation of a peptide bond N-terminal to the isoAsp exceeding the threshold is indicative of the presence of isoAsp in a protein. In various embodiments, the mass spectrometry system is MALDI-TOF/TOF. In various embodiments, the mass spectrometry system is LC-MS/MS. In various embodiments, the mass spectrometry system uses higher energy collisional dissociation (HCD).

### Protein Sample

The protein sample (which, for conciseness, may also be referred to herein simply as a "protein") comprises any type of protein that may be processed or analyzed for stability and/or structural integrity. In various embodiments, the protein sample so subjected to the methods disclosed herein, comprises or consists of a large peptide, antibody, antibody fragment, antibody fusion peptide or antigen-binding fragments thereof. In related embodiments, the antibody is a polyclonal or monoclonal antibody.

### Quality & control

Aspartic acid (Asp) to isoaspartic acid (isoAsp) isomerization in therapeutic monoclonal antibodies (mAbs) and other biotherapeutics is a critical quality attribute (CQA) which requires careful control and monitoring during the discovery and production processes. The unwanted formation of *iso*Asp within biotherapeutics and resultant structural changes in the peptide backbone may negatively impact the molecule activity or become immunogenic, especially if the isomerization occurs within the complementarity determining region (CDR).

The methods disclosed herein may be used to monitor *iso*Asp during the protein discovery (Research) process or a protein production (Process Development) process. In various embodiments, an absence of the *iso*Asp indicates suitability of the protein for further processing. In various embodiments, the presence of *iso*Asp may result in one or more of the following changes to the protein: structural changes in the peptide backbone, change the protein activity, or increase the immunogenicity. In various embodiments, the presence of *iso*Asp results in structural changes in the peptide backbone compared to a reference protein comprising an L-Asp at the same position. In some embodiments, the presence of *iso*Asp inhibits potency of the protein. In various embodiments, the presence of *iso*Asp increases the immunogenicity of the protein compared to a reference protein comprising an L-Asp at the same position. As described herein, protein (e.g., a sample, clone, lot, or batch comprising the protein) may be rejected if the protein contains excessive amount of *iso*Asp (typically about 5% in the Research Discovery stage)

In various embodiments, rejecting the protein comprising the *iso*Asp comprises rejecting a quantity of product comprising the protein, or rejecting a clone that produces the protein. In various embodiments, the method further comprises reducing or inhibiting *iso*Asp by protein engineering. In various embodiments, the method further comprises engineering the protein to remove the site that is susceptible to aspartate isomerization comprises inducing a point mutation at the position of a nucleic acid encoding the *iso*Asp in the protein. In some embodiments, the protein comprising the isoAsp is rejected if the isoAsp is at or above a specified level.

### Aspartic acid (Asp) to isoaspartic acid (isoAsp) isomerization

Aspartic acid (Asp) to isoaspartic acid (*iso*Asp) isomerization in therapeutic monoclonal antibodies (mAbs) and other biotherapeutics is a critical quality attribute (CQA) which requires careful control and monitoring during the discovery and production processes. The unwanted formation of isoAsp within biotherapeutics and resultant structural changes in the peptide backbone may negatively impact the molecule activity or become immunogenic, especially if the isomerization occurs within the complementarity determining region (CDR).

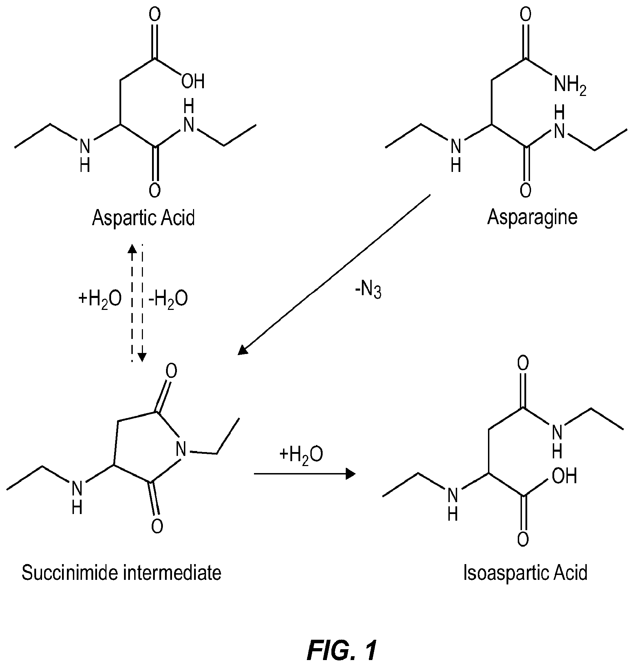

Characterization of *iso*aspartate (*iso*Asp) formation in a protein by canonical mass spectrometry (MS) can be a formidable task because the modified residue is isomeric with its unmodified counterpart¹⁹. The chemistry of *iso*Asp formation has been well documented (Figure 1) and proceeds via the formation of a cyclic imide intermediate, hydrolysis of which results in the generation of both *iso*Asp and Asp. Depending on the actual amino acid sequence and the protein structure, the ratio of the former to the latter is usually about 3:1 and isomerization is favored if the Asp is followed by a small residue such as a glycine¹⁹. Racemization at the aspartate residue is also possible during the process, resulting in the formation of both D-*iso*Asp and D-Asp. However, racemization is relatively rare and racemized amino acids have been detected in long-lived proteins such as lens crystallins and tooth dentine^{20-21,22}. Repairing *iso*Asp residues in modified cellular proteins is crucial and an enzyme that partially converts *iso*Asp residues in proteins (protein L-*iso*aspartyl methyl transferase PIMT) back to aspartate has been identified in microbial, mammalian and plant systems²³⁻²⁴. A detailed review on the biology and the substrate specificity of this enzyme has been published²⁵.

Because of the additional methylene group introduced into the polypeptide backbone and the concomitant shortening and reorientation of the carboxyl side chain²⁶, this structural change may impact the protein's function, becoming immunogenic²⁷ or leading to unwanted aggregation and resulting in insolubility²⁶. Isomerization of Asp1 and Asp7 in β-amyloid (Aβ 1-42) was first identified in Alzheimer's Disease (AD) brain tissue and has been implicated to be responsible for initiating the pathological effect during the development of the disease. In a recent study, Julian and associates²⁹ demonstrated Aβ with *iso*Asp7 modification was resistant to proteolysis by the cathepsins commonly found in lysosomes. The authors also proposed an association between this lysosomal storage malfunction and the development of AD.

*Iso*Asp can be identified by automated Edman degradation where the Edman chemistry would terminate at the modified residue⁴⁰. However, N-terminal sequencing is time consuming, requiring a larger amount of material when compared to LC-MS/MS. Lehmann⁴¹ reported the use of LC-MS/MS to analyze *iso*Asp in peptides and demonstrated that complementary b/y ion intensity ratios obtained by fragmentation of the (Asp/*iso*Asp)-X bond could be employed to distinguish between the modified residue from its unmodified counterpart. However, the MS/MS data presented by Bondarenko and co-workers⁴² suggested the observation made by Lehmann, may not be applicable to all *iso*Asp containing peptides. Identification of *iso*Asp in peptides by MALDI-Post Source Decay (PSD) has been reported by Fujii⁴³ and Reilly⁴⁴. By this technique, the former confirmed the detection of the isoAsp diagnostic ion yL-n+1 - 46 (where L represents the length of peptide (integer; total residue number), n is the position of the isomerized residue) first reported by Gonzalez⁴⁵. Unfortunately, this ion is usually of very low intensity and is generally limited to peptides obtained by tryptic digestion. This group also evaluated the ratio yL-n: yL-n+1 from the PSD fragmentation of a series of tryptic-like synthetic peptides based on the sequence of crystallin. In general, for the isoAsp containing peptide, the intensity of the yL-n+1 is increased compared to a corresponding peptide containing L-Asp. The authors attributed this observation to the shorter isoaspartyl side chain interacting with the carbonyl group in the peptide bond N-terminal to the modified residue. Fuji et al. utilized Post source decay-matrix assisted laser desorption ionization (MALDI-PSD) to evaluate the amount of isoAsp formation from a tryptic digest of human aged lens crystallin; a relatively small protein. In contrast, the methods disclosed herein are able to detect isoAsp in larger peptides and proteins with consecutive aspartate residues.

Detection of *iso*Asp diagnostic ions using either ECD or ETD (c' + 57 and z•-57) has been reported^{16, 45-48}. In contrast, the instant disclosure describes fragmentation of the singly charged ion that allows *iso*Asp modification to be distinguished from its unmodified counterpart (Asp). Depending on the peptide sequence, the signature ions can be of low intensity, thus making interpretation challenging. *Iso*Asp containing peptides derived from β-2 microglobulin have also been evaluated by Costello⁴⁹ using MALDI-ISD (in-source decay). In the presence of hydrogen donating matrix, the ECD-like c' + 57 and z•-57 diagnostic ions were also observed. The use of radical directed dissociation (RDD) in identifying amino acid isomers was described by Julian. The RDD method has been successfully demonstrated in identifying isoAsp formation and epimerization of serine and other amino acids⁵⁰⁻⁵¹. Smith⁵² reported separation of four synthetic derivatives of β-amyloid peptides (residues 6-16) with Asp7 being unmodified, replaced with D-Asp, L-*iso*Asp or D-isoAsp using SLIM (structures for lossless ion manipulations) instrumentation. Studies by Cooks⁵³ demonstrated the use of ion chemistry to distinguish Asp and *iso*Asp containing peptides. The carboxylate side chain was modified with carbodiimide to obtain acylisourea. Subsequent 1,3-acyl shift led to the formation of N-acylurea. Under collision induced dissociation (CID) conditions, distinct fragments of acylisourea and N-acylurea can be detected. However, because of steric hindrance imposed by the *iso*Asp side chain, formation of N-acylurea is not favored. Thus, the predominant fragment observed with the modified peptide was that derived from acylisourea. In addition, Popov⁵⁴⁻⁵⁵ reported using MALDI-TOF/TOF with CID to explore fragmentation of Aβ (residues 1-42) containing *iso*Asp7. By using a series of synthetic peptide standards, these investigators developed a label-free quantitation method of Aβ. However, these investigators do not appear to mention identification of *iso*Asp or mass spectra for identifying isoAsp itself. Moreover, it is not expected that the methods reported by these investigators would have detected consecutive isoAsp in peptides with consecutive aspartate residues.

In various embodiments, the methods disclosed herein may be used to determine the position of *iso*Asp residues in a protein. In addition, the disclosed herein may be used for quality control during the protein production process. The disclosed method may also be used to assess stability or structural stability of the protein after storage or exposure to temperature changes, light or other environmental or chemical factors.

The following examples are given merely to illustrate the present invention and not in any way to limit its scope.

### EXAMPLES

### General Methods

Nine pairs of synthetic peptides, comprising either Asp or isoAsp, were obtained from Anaspec (Freemont, CA) or synthesized in-house. The peptide sequences are: LD/*iso*DA, GD/*iso*DLLLK , GLD/*iso*DLLK , ALD/isoDGK , ALD/*iso*DEK , ALD/isoDGE , AD/*iso*DLGK , GFYPSDIAVEWESD/*iso*DGQPENNYK and VVSLTVLHQD/*iso*DWLNGK . The delta-sleep inducing peptides DSIP and β-Asp DSIP (WAGGDASGE (SEQ ID NO: 2) and WAGGisoDASGE (SEQ ID NO: 1) were purchased from Bachem (Torrance, CA). The MS grade proteolytic enzymes chymotrypsin and trypsin were purchased from Thermos Scientific (Waltham, MA). Dithiothreitol (DTT) and iodoacetamide (IAM) were obtained from Sigma Aldrich (St Louis, MO). The MALDI matrix used (4-HCCA) was from Bruker Daltonics (Billerica, MA). All other reagents used were of the highest grade commercially available. Optima LC-MS grade solvents were used throughout.

### Thermal Stressing and Proteolytic Degradation of Commercial Therapeutic mAb

The therapeutic mAb, at a concentration of 1.26 mg/mL was incubated in 0.1 M ammonium bicarbonate (pH 7.8) at 40°C for 2 weeks to induce aspartate isomerization. The sample was reduced (DTT) and alkylated (IAM) prior to digestion with trypsin or chymotrypsin and subsequent LC-MS/MS analysis.

### MALDI-TOF/TOF Analysis of Selected Peptides

MALDI-MS was performed by using a Bruker UltrafleXtremeTOF/TOF mass spectrometer, equipped with a 2,000 Hz smartbeam II (Nd:YAG) laser. 4-HCCA was used as the matrix (saturated solution in 0.1 % TFA/50% acetonitrile).

### Peptide Mapping by LC-MS/MS

The column used was an Acquity UPLC peptide BEH C18 (2.1 x 150 mm, 1.7 µm) column. Solvent A was 0.1 % formic acid in water and solvent B was 0.1 % formic acid in acetonitrile. The column was equilibrated in 1 % B at a flow rate of 0.25 mL/min and at 50°C. Upon sample application, the column was washed with 1 % B for 8 min before a linear gradient from 1 to 55 % B over 70 min was applied. Peptide elution was monitored by absorbance at 214 nm and the eluted peptides were introduced into a Q-Exactive for MS and MS/MS. A spray voltage of 3.5 kV was used. The data-dependent MS method consisted of a full MS from m/z 300-2000 at a resolution of 70,000. This was followed by HCD (at resolution 17,500) of the 10 most abundant precursors. AGC target values were set at 1E6 for full MS and 5e4 for HCD. An isolation width of 2 Da and a NCE of 27 were employed. The tandem MS data obtained was analyzed by for peptide identification.

### EXAMPLE 1: Identification of IsoAspartate in peptide standards

Within the current literature, the major application of MALDI-MS has shifted towards imaging⁵⁶⁻⁵⁷, identification of bacterial species⁵⁸ and high throughput screening of small molecules library⁵⁹⁻⁶¹. The LIFT-TOF/TOF technology introduced almost two decades ago has demonstrated its successful application in peptide characterization⁶² . Briefly, as opposed to the slow heating process as a function of the low energy ion-neutral collision that is CID⁶³ , the LIFT technology is based on the result of metastable ion fragmentation in the field-free region of the MS, followed by precursor and fragment ion gating prior to energy focusing in a reflectron⁶⁴.

Figure 2A & 2B provide the MALDI-TOF/TOF data obtained for tripeptides L*iso*DA and LDA respectively. For the LisoDA peptide, the predominant ion (y₂) demonstrated fragmentation at the bond N-terminal to the isoAsp residue is preferred. Additionally, the aspartyl peptide clearly demonstrated that the major cleavage site was after the aspartate residue with the generation of b₂ ion, which is attributed to the "aspartate-effect" ⁶⁷. The 2 peptides were mixed together in defined proportions of L*iso*DA and LDA prior to being subjected to identical MALDI TOF/TOF fragmentation and the ratio of y₂/b₂ was measured (Figure 2C). A plot of the ratio of the two ions against %-*iso*aspartate peptide in the binary mixture resulted in a curve (y = 0.2188e0.023x ; Figure 2C, with R2 >0.99) rather than a straight line. The results obtained from another pair of *iso*D/D peptides (WAGG*iso*DASGE (SEQ ID NO: 1) and WAGGDASGE (SEQ ID NO: 2) are shown in Figures 2D & 2E. Examination of the *iso*Asp peptide MALDI-TOF/TOF spectrum shows the enhancement in y₅ ion was readily observed when compared to the unmodified peptide, again demonstrating the preferential cleavage N-terminal to the *iso*Asp residue. The b₅ ion would be derived from cleavage at the C-terminal side of *iso*D/D. The ratio of y₅/b₅ was measured for a series of well-defined of mixture of *iso*D/D peptide. A plot of the ratio against the %-*iso*Asp in the binary mixture is again a curve with y = 0.2039e0.019x and R2 > 0.99 (Figure 2F).

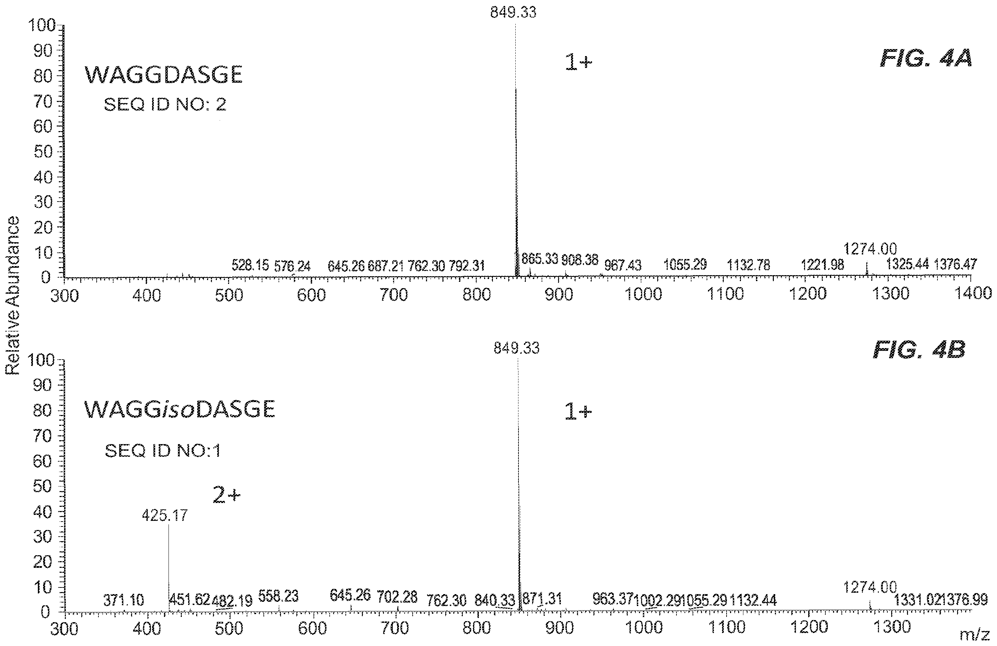

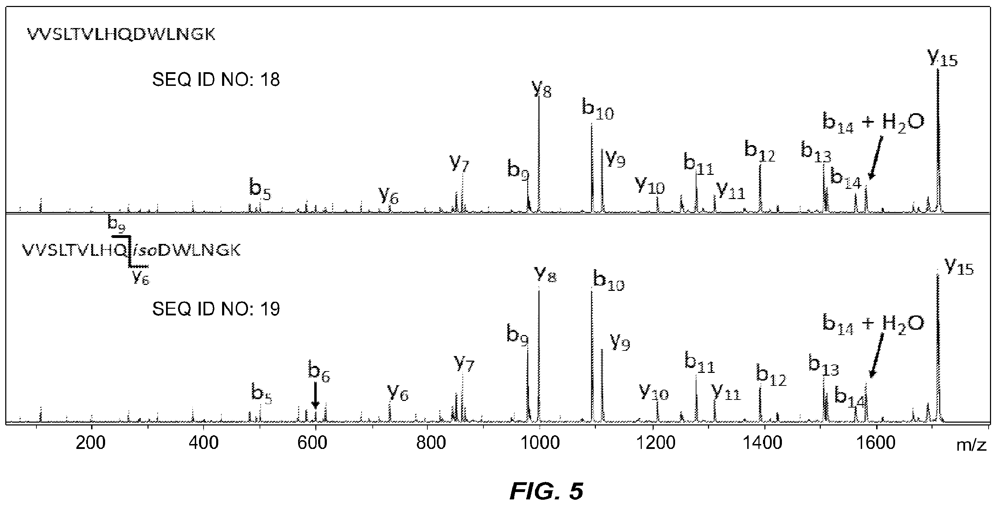

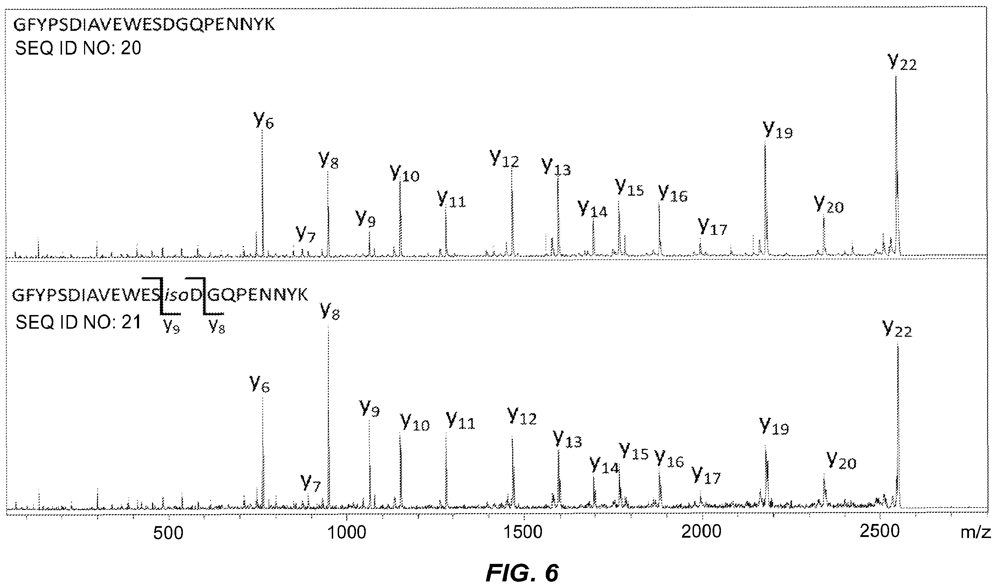

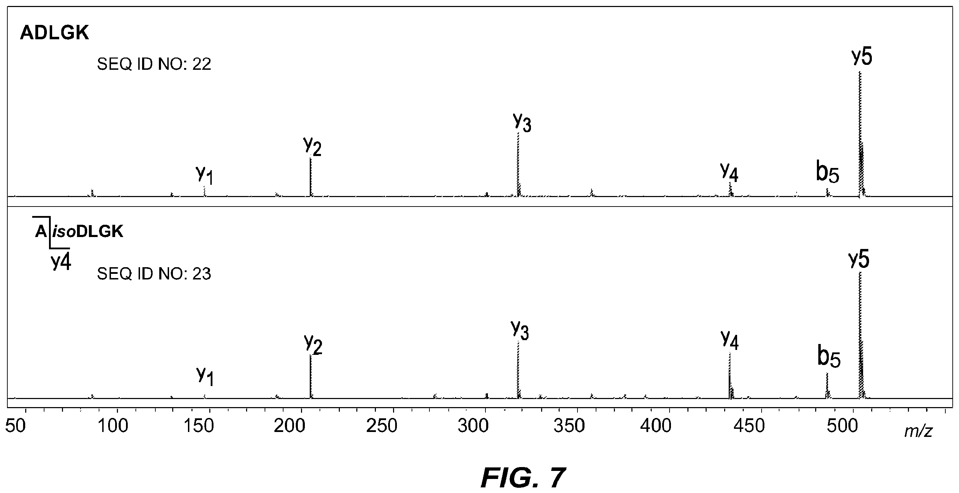

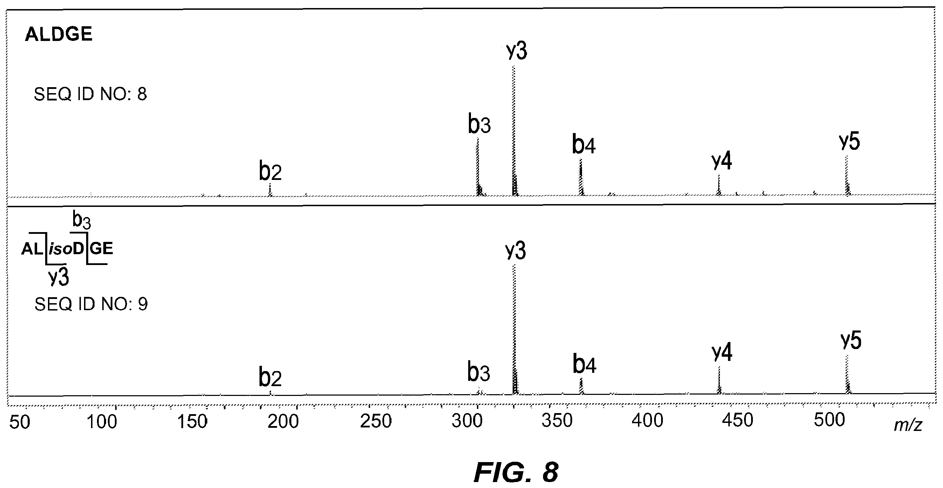

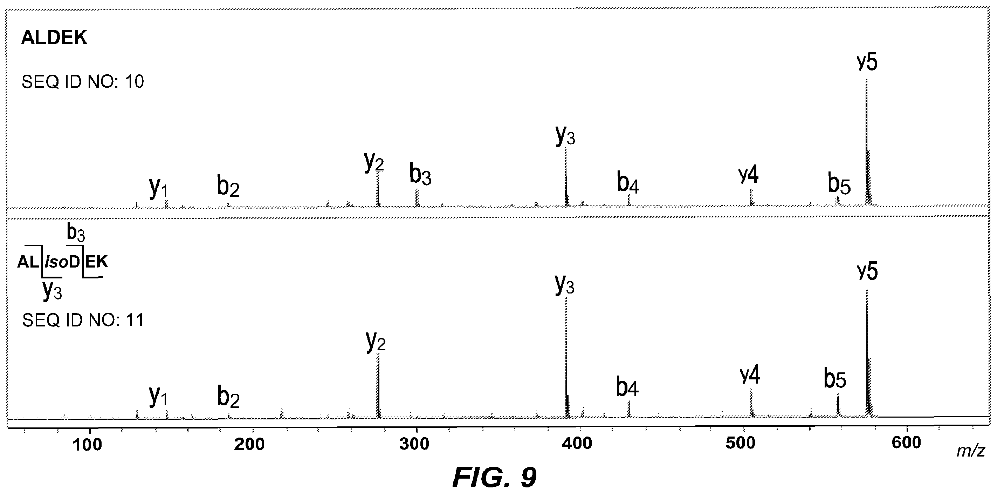

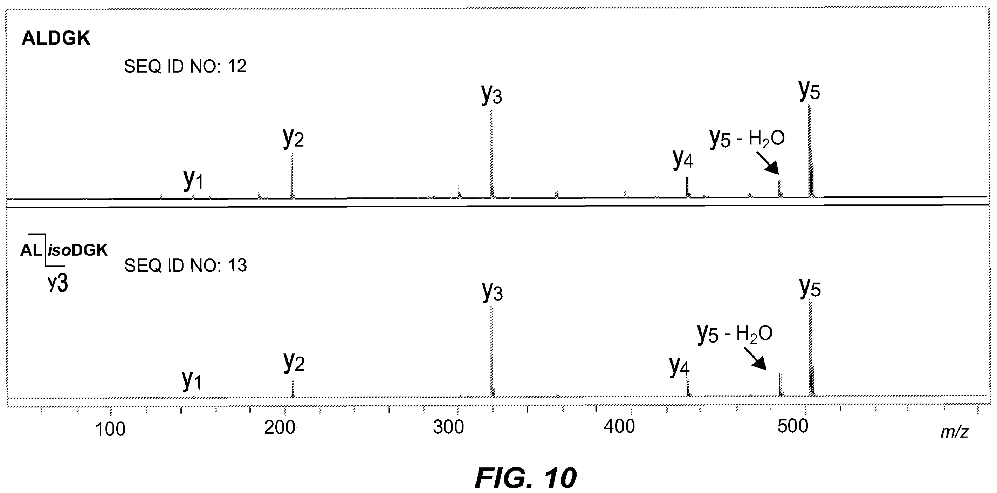

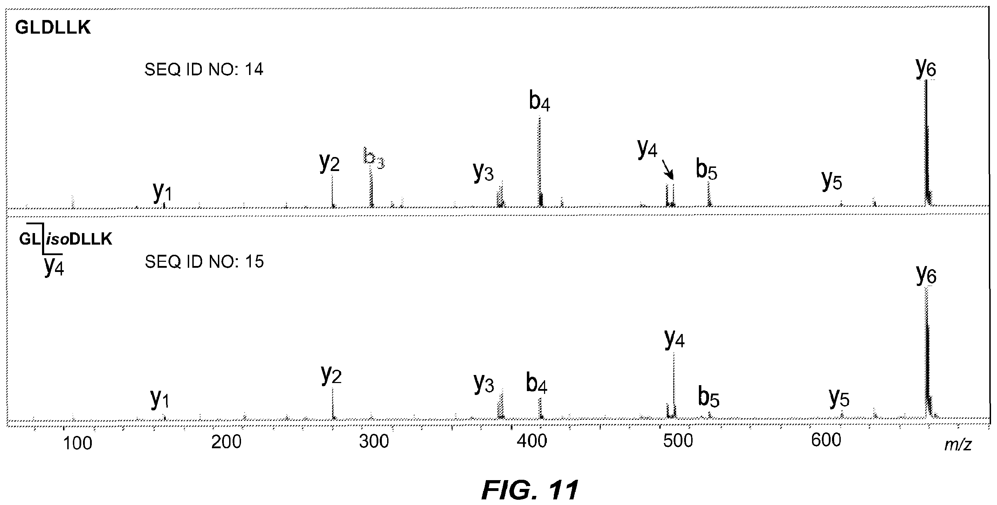

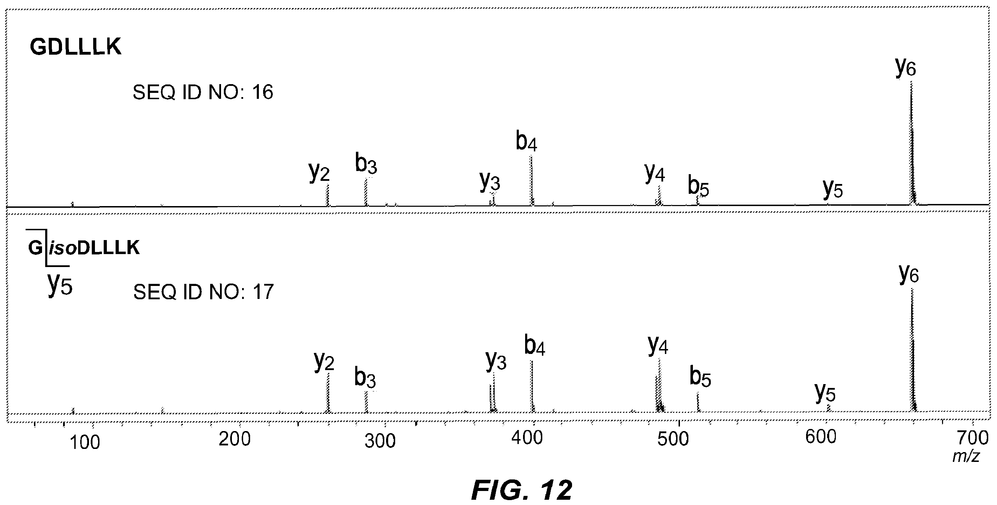

A similar observation was made by Indeykina⁶⁸ in their tandem-MS study on a binary mixture of synthetic amyloid-β (residues 1-16) peptides containing either Asp7 or *iso*Asp7. It is likely that the *iso*Asp containing peptide is more easily ionized. In addition, upon ESI infusion of the 2 peptides WAGG isoD/D ASGE (Figures 4A-4B) the *iso*Asp containing peptide displayed both [M+H]+ and [M+2H]2+ species, whereas the aspartate containing peptide only displayed the singly charged species [M+H]+, suggesting that the proton affinity for the *iso*Asp containing peptide is greater or the ionic structure is sterically favorable to accept a second proton. However, under identical conditions, the doubly charged species was readily detected in the *iso*D peptide. Multiple other *iso*Asp/Asp containing peptide standards were evaluated by MALDI-TOF/TOF are shown in Figures 5 to 12, all of which demonstrate preferential peptide bond fragmentation immediate N-terminal to the isoAsp residue.

### EXAMPLE 2: Identification of IsoAspartate in Thermal Stressed mAb

To put the above observations into the context of a real-life therapeutically relevant mAb, the MALDI-TOF/TOF method was used to identify an isomerized Asp residue in a mAb after thermally induced forced degradation. It has been well documented that a commercial mAb has an Asp residue at position 102 in the CDR3 domain of its heavy chain that has the propensity to undergo isomerization and modification of which results in loss of activity^{37,69}.

In this example, the mAb was incubated in pH 7.8 and at 40°C for 2 weeks followed by reduction, alkylation and tryptic digestion. An unstressed mAb treated under identical conditions was used as control. Upon thermal stressing, other modifications such as Asn deamidation and Met oxidation were also present. Because they have been previously identified and documented by others⁷⁰, Asn deamination and Met oxidation were not examined in these sets of experiments. Therefore, this disclosure describes characterization of heavy chain CDR3 Asp102 isomerization.

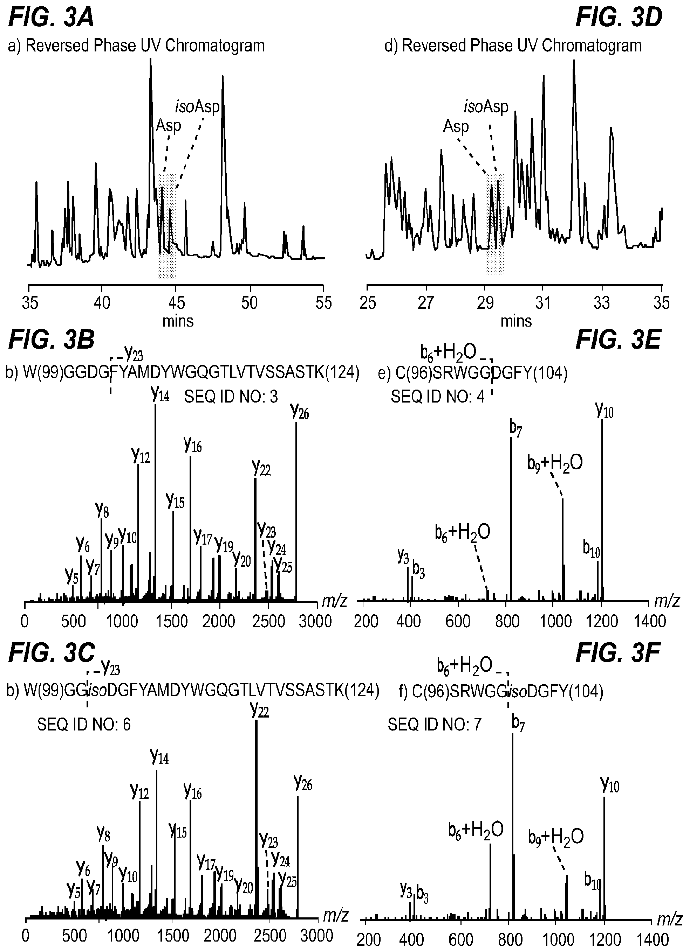

Figure 3A shows the LC-UV tryptic map of the thermal stressed mAb (zoomed in to the region of interest). Analysis of the LC-MS/MS data demonstrated the tryptic peptide encompassing heavy chain Asp102 (W99GGDGFYAMDYWGQGTLVTVSSASTK124; SEQ ID NO: 3) were eluted in two well resolved peaks (Figure 3A) with identical *m*/*z* values, 1392.63 ([M+2H]2+). The ratio of the first peak to the second peak is approximately 1:1. The LC-MS/MS CID data of the doubly charged ions were identical and the earlier eluted peak is typically assumed to contain the modified residue (*iso*Asp) based on the data reported in the current literature⁴⁰. In the control protein digest, the later eluted peak was minimal (data not shown). The MALDI-TOF/TOF results for the two tryptic peptides are shown in Figures 3B & 3C where the former is from the earlier eluted peak (44.1 mins) and the latter is from the later eluted peak (44.7 mins). The enhancement in y₂₃ was readily observed in Figure 3C, showing fragmentation N-terminal to Asp102 was increased, hence indicating the residue has isomerized. The ratio of the two fragments y₂₃ and y₂₄ of each peptide was determined and the Rᵢₛₒₘₑᵣ⁵⁰ of the two peptides was calculated to be 2.04. For this peptide, the increase in the intensity of the y₂₂ ion shows the peptide bond C-terminal to the isoAsp side chain was more labile to fragmentation under the conditions employed. The data therefore demonstrates the order of elution from a RP-HPLC column cannot be used to definitively identify the isoAsp containing peptide from its unmodified counterpart, as in this case, the isoAsp containing peptide was eluted later.

Because the tryptic peptide was large (26 residues) an experiment was therefore carried out to evaluate if a shorter peptide would have an improved MALDI-TOF/TOF spectrum. Both the thermal stressed and control commercial mAb were digested with chymotrypsin after reduction and alkylation and the peptides were identified by LC-MS/MS. The decapeptide C96 SRWGGDGFY104 (SEQ ID NO: 4) again was found to resolve into two chromatographically separated peaks with identical *m*/*z* = 602.73 ([M+2H]²⁺; Figure 3D). Because of the presence of Arg at residue 98 in the peptide, the fragment ions were dominated by the b-ion series. A side by side comparison of their fragmentation data in Figures 3E & 3F show a prominent b₆ + H₂O in Figure 3F, demonstrating with the later eluted peak, fragmentation of the peptide bond N-terminal to the modified residue was enhanced. Thus, it can be concluded that the later eluted peptide was the isoAsp containing peptide. By determining the ratio of two fragments, in this case the b₆ + H₂O and b₁₀ of each peptide, Rᵢₛₒₘₑᵣ was calculated to be 11.0. The mechanism for the addition of water as indicated by the detection of b₆ + H₂O ion has been examined⁷¹ and has been pproposed to be due to molecular rearrangement from the isoAsp carboxylic acid side chain.

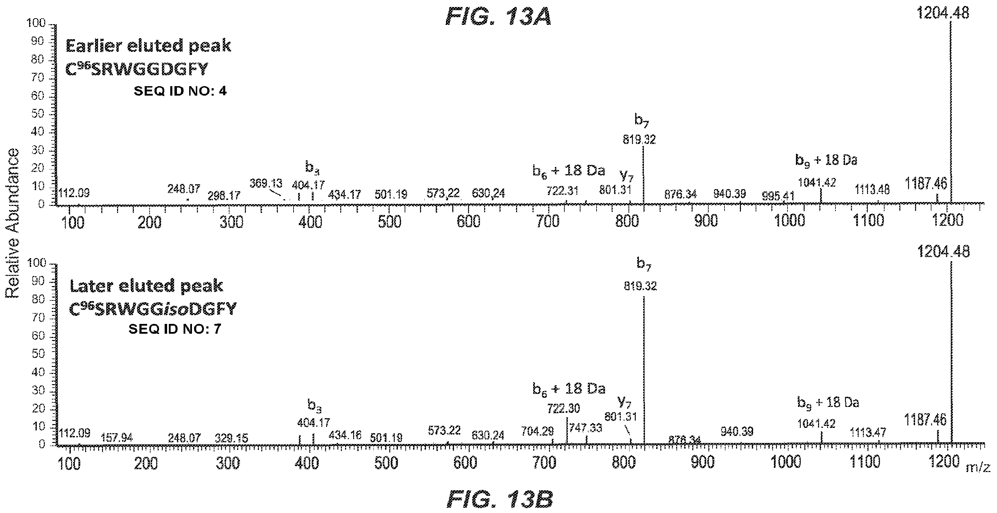

Initially, it was unclear how only the MALDI-TOF/TOF was successful in identifying isoAsp containing peptide but not LC-MS/MS. During identification of the chymotryptic peptides by LC- MS/MS, HCD analysis of the singly charged C96-Y104 was observed to produce a highly comparable spectrum to the MALDI TOF/TOF data, that is, a predominant b₆ + H₂O ion, indicative of Asp102 being the isomerized residue (Figure 13). However, for larger peptides, such as the 26-residue tryptic peptide (Figures 3B & 3C; tryptic digest), it could be difficult to examine the singly charged ion by ESI-HCD because the precursor ion would typically be a doubly or triply charged species. Thus, LC-MS/MS could be used to identify potential aspartate isomerization sites, the only caveat being the region of the protein containing the chemical liability must be cleaved into smaller peptides, likely by a non-specific protease, so that the singly charged ion can be subjected to MS/MS HCD fragmentation. It is noted that in a study on Asn10 deamidation in Stem Cell Factor⁷², MS/MS of the hexapeptide (VTisoDNVK; SEQ ID NO: 5) produced an intense y₄ ion when compared to the unmodified or the Asp containing peptide. The authors only attributed this observation to be due to an unusual cleavage of the T-isoD bond. Alternatively, charge reduction⁷³ could be utilized to reduce peptide charge states to z = 1+, therefore potentially allowing for efficient Asp/isoAsp identification by the more ubiquitously used LC-MS/MS techniques.

### EXAMPLE 4: Thermal Stressing and Proteolytic Degradation of Commercial Therapeutic Antibody

The antibody, at 1.26 mg/mL was incubated in 0.1 M ammonium bicarbonate (pH 7.8) at 40°C for 2 weeks prior to being frozen at -70°C for further experiments. In a typical digestion, 100 µL of the incubated antibody was dried by vacuum centrifugation. The sample was dissolved in 50 µL of 8 M urea in 0.1 M Tris at pH 7.5 before being reduced with 10 mM DTT at 50°C for 1 hr and followed by alkylation with 20 mM iodoacetamide in the dark for 30 min at room temperature. Alkylation was quenched by the addition of DTT. The sample was diluted with 150 µL of 0.1 M Tris at pH 7.5 and either digested with 1 % by weight of trypsin or chymotrypsin. Another aliquot of the antibody that has not been subjected to thermal stressing was treated in an identical fashion served as the control. Another set of heat stressed experiments was performed by incubating the antibody in pH 5.2. The amount of heavy chain Asp102 isomerization was similar and only the data at pH 7.8 was provided here.

### EXAMPLE 5: MALDI-TOF/TOF Analysis of Selected Peptides

MALDI-MS was performed by using a Bruker UltrafleXtremeTOF/TOF mass spectrometer, equipped with a 2,000 Hz smartbeam II (Nd:YAG) laser. 4-HCCA was used as matrix (10 mg/mL in 0.1 % TFA/50% acetonitrile). 1 µL of peptide was mixed with an equi-volume of matrix before 1 µL of the mixture was spotted on an MTP 384 ground steel target plate, air-dried and inserted into the instrument for mass measurement. The mass spectrometer was set in the positive mode using a 21kV acceleration voltage and a 25 kV reflectron voltage. The laser power was optimized and in a typical experiment, 5,000 laser shots were accumulated to create the spectra. Fragmentation of selected peptides was recorded using the LIFT cell technology. An initial acceleration voltage of 7.5 kV and a LIFT cell acceleration voltage of 19 kV were employed. Laser power for both the precursors and the fragments were optimized. Usually, about 8,000 to 10,000 laser shots were used in recording the fragmentation data.

The MALDI-TOF/TOF data of 8 pairs of synthetic peptides are further illustrated in Figures 5 to 12. In all cases, with the *iso*D containing peptide, the ion generated by fragmentation of the peptide bond N-terminal to the modified residue wasmore intense. The Rᵢₛₒₘₑᵣ values range from 1.5 to 5.8. The technique can therefore be readily employed in distinguishing an isoD containing peptide from its unmodified counterpart.

In summary, the data provided herein demonstrates that fragmentation using MALDI-TOF/TOF provides a simple and robust, label-free analytical method to identify the location of the *iso*Asp residue. This was demonstrated in peptide standards and a thermally degraded therapeutically relevant mAb. The peptide bond N-terminal to the *iso*Asp residue was demonstrated to be more labile to fragmentation by PSD. LC-MS/MS HCD fragmentation of a singly charged (*z* = 1+) isoAsp containing peptide produces MS/MS fragmentation data highly comparable to MALDI-TOF/TOF fragmentation data. Additionally, the potential for online LC-identification has been discussed herein, by the implementation of charge reduction followed by LC-MS/MS (HCD), but again, relying on chromatographic separation for unequivocal identification. Online LC-MS/MS identification, as used herein, refers to utilizing electrospray ionization and online chromatographic separation of digested peptides followed by MS and MS/MS detection and fragmentation respectively. The method disclosed herein is improved using online LC-MS/MS identification because the manual collecting step can be skipped. Collecting the chromatographically separated peptides of interest manually is time-consuming. Both targeted LC-MS/MS fragmentation of the z = 1+ ion and charge reduction could have important implication if applied to late stage Process developments analytical methods, such as the Multi Attribute Method⁷⁴.

A potential limitation of this MALDI-TOF/TOF method is that the Asp and isoAsp containing peptide must display chromatographic separation, therefore allowing collection and subsequent MALDI-TOF/TOF analysis. However, if chromatographic separation can be achieved, isoAsp determination is straight forward and unequivocal.

### EXAMPLE 6: Identification of aspartate isomerization in a sample of heat stressed antibody protein product

The following example describes experiments performed to detect *iso*Asp in a sample of heat stressed antibody protein product comprising adjacent isoaspartic acid and L-aspartic acid.

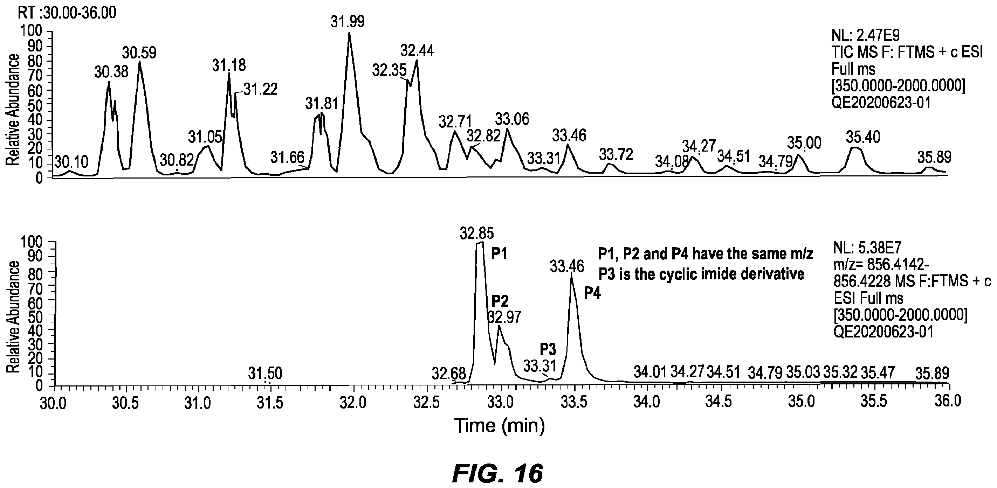

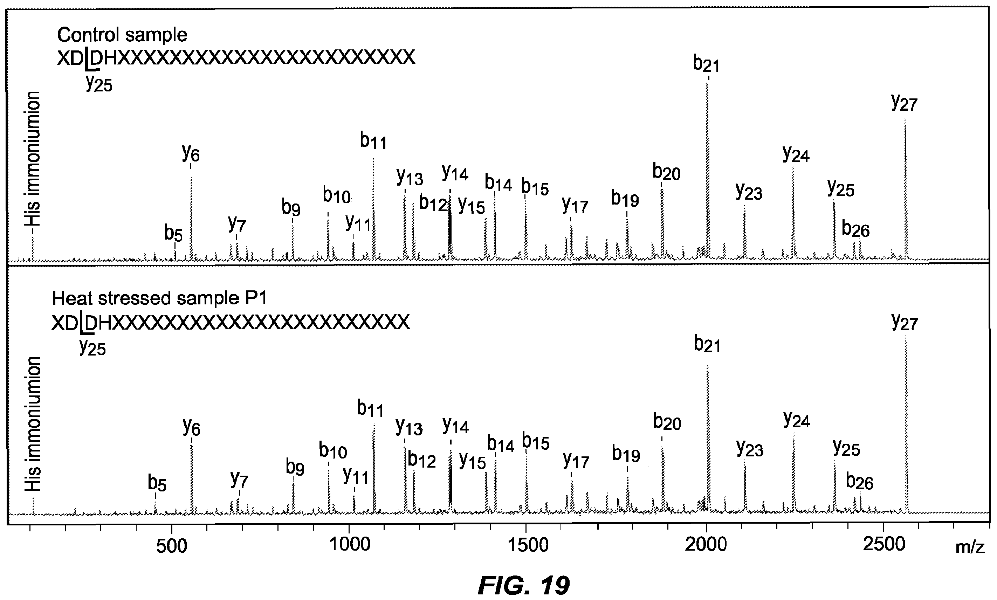

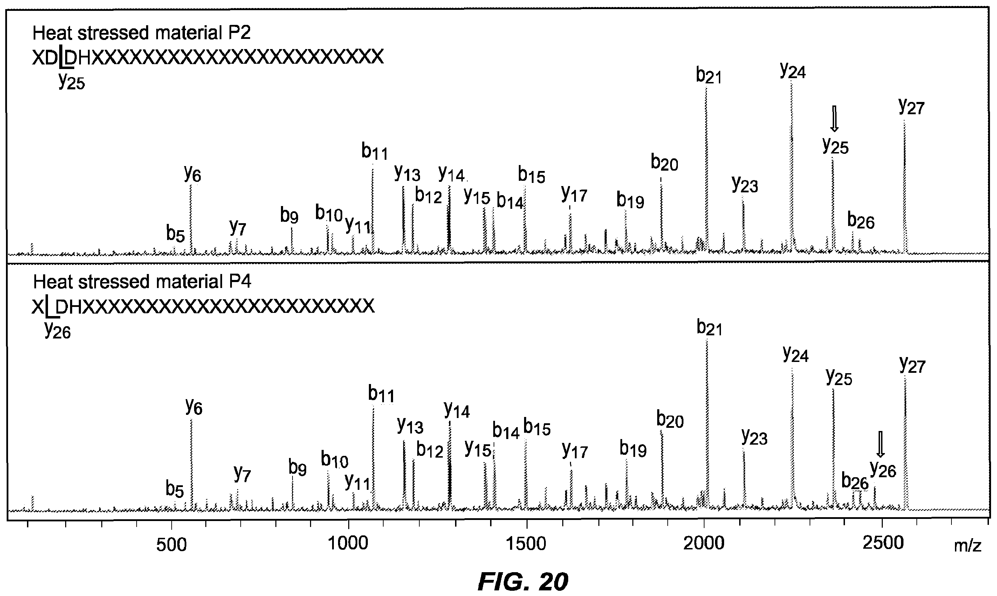

A sample of thermal stressed antibody protein product (3 weeks at 40°C) was reduced, alkylated with iodoacetamide and digested with trypsin as described above in Example 4. The digest was subsequently analyzed by LC-MS/MS (Tryptic mapping Filename: QE20200623-01). Analysis of the spectrum revealed two main peaks and a small shoulder (Fig. 16). A peak with mass corresponding to the succinimide derivative was also detected. The peaks were labeled as P1 to P4 with P3 corresponding to the cyclic imide (Fig. 16). An aliquot was then manually collected and the peptides of interest corresponding to XDDHXXXXXXXXXXXXXXXXXXXXXXX were identified and analyzed by MALDI-TOF/TOF. P1 was determined to be the unmodified peptide (Fig. 19), P2 (the shoulder) was identified as the XDisoDHXXXXXXXXXXXXXXXXXXXXXXX (Fig. 20), P4 was identified as X*isoD*DH XXXXXXXXXXXXXXXXXXXXXXX (Fig. 20). In both unstressed and stressed P1, the ratio of the intensity of y₂₅ to y₂₃ is approximately 1:1 (Fig. 19), indicating P1 is the remaining unmodified peptide. With P2, y₂₅ to y₂₃ was determined to be 1.7 to 1, indicating the modification is at isoDH. With P4, the readily detectable y₂₆ demonstrates the modification is at isoDD (Fig 20). The proposed peaks were confirmed with synthetic peptide. Thus, it has been observed that methods as described herein can resolve an *isoAsp* residue that is adjacent to a L-Asp residue in the primary sequence of a peptide.

The Orbitrap Fusion was used to specifically fragment the 1+ species of the peptide and to adapt EThcD as a routine method for isoAsp identification.

### EXAMPLE 7: Evaluation of two synthetic peptides with the modified residue proximal to the C-terminus

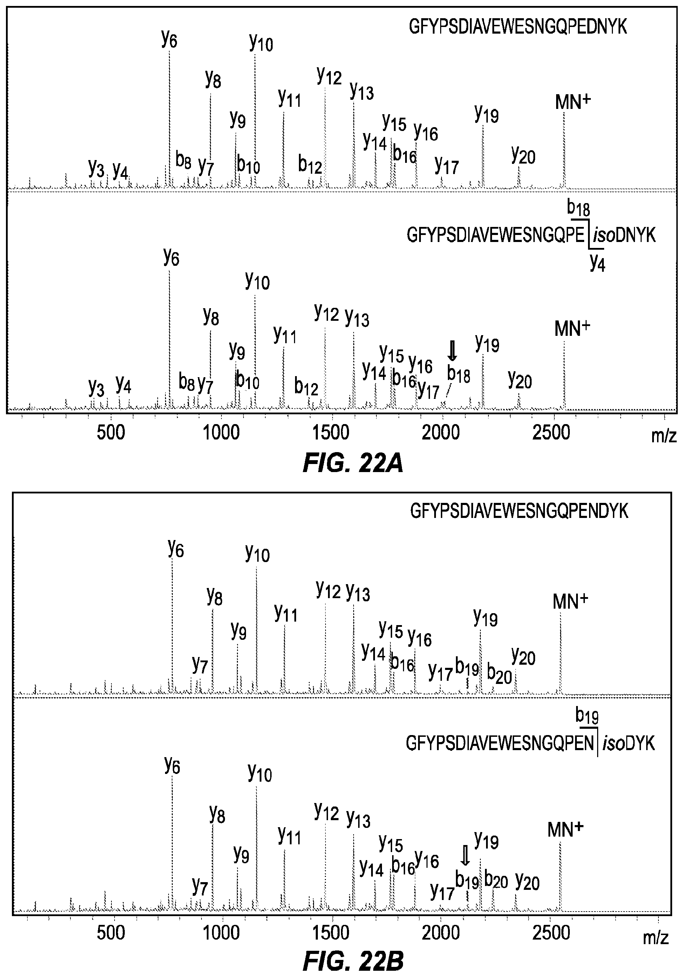

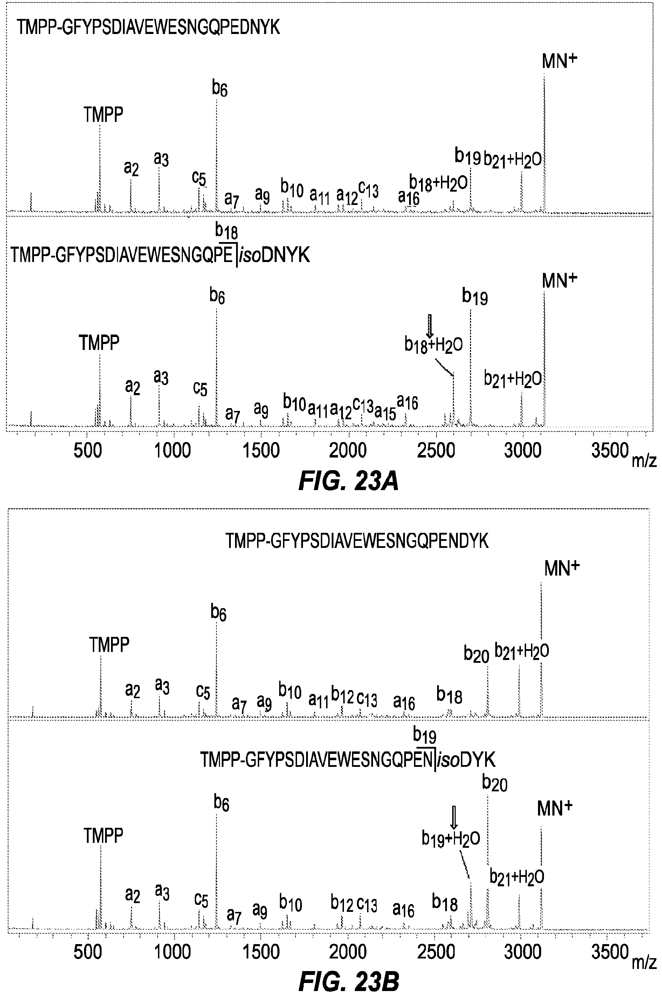

Evaluation of 2 synthetic peptides with the modified residue proximal to the C-terminus (GFYPSDIAVEWESNGQPENisoDYK (SEQ ID NO: 27) & GFYPSDIAVEWESNGQPEisoDNYK (SEQ ID NO: 25)) by the MALDI-TOF/TOF method described in Example 1 shows that while there is an increase in fragmentation of the peptide bond N-terminal to the modified residue, the enhancement was of smaller magnitude, thus making data interpretation more challenging (Figures 22A & 22B). However, after introduction of a positive group to the N-terminus, such as chemical modification using the reagent (N-succinimidyloxycarbonylmethyl) tris (2,4,6 trimethoxyphenyl) phosphonium bromide (TMPP), MALDI-TOF/TOF fragmentation of the resultant isoAsp containing peptide gave an intense bₙ₋₁+H₂O (where n is the residue number of the isoAsp in the peptide) when compared to the Asp containing counterpart (Figures 23A & 22B). The transfer of water was from the shortened *iso*Asp carboxyl side chain, which has been documented. Thus, modification of the N-terminus with a N-terminal charge-derivatizing reagent such as TMPP followed by MALDI-TOF/TOF allows a rapid and unequivocal identification of the isoAsp residue even if it is proximal to the C-terminus.

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

### References

1. Guccione, E.; Richard, S., The regulation, functions and clinical relevance of arginine methylation. Nat Rev Mol Cell Biol 2019, 20 (10), 642-657.
2. Pawson, T.; Scott, J. D., Protein phosphorylation in signaling--50 years and counting. Trends Biochem Sci 2005, 30 (6), 286-90.
3. Deol, K. K.; Lorenz, S.; Strieter, E. R., Enzymatic Logic of Ubiquitin Chain Assembly. Front Physiol 2019, 10, 835.
4. Moremen, K. W.; Tiemeyer, M.; Nairn, A. V., Vertebrate protein glycosylation: diversity, synthesis and function. Nat Rev Mol Cell Biol 2012, 13 (7), 448-62.
5. Holdener, B. C.; Haltiwanger, R. S., Protein O-fucosylation: structure and function. Curr Opin Struct Biol 2019, 56, 78-86.
6. Zachara, N. E.; Hart, G. W., Cell signaling, the essential role of O-GlcNAc! Biochim Biophys Acta 2006, 1761 (5-6), 599-617.
7. Zachara, N. E., Critical observations that shaped our understanding of the function(s) of intracellular glycosylation (O-GlcNAc). FEBS Lett 2018, 592 (23), 3950-3975.
8. Walsh, C. T.; Garneau-Tsodikova, S.; Gatto, G. J., Jr., Protein posttranslational modifications: the chemistry of proteome diversifications. Angew Chem Int Ed Engl 2005, 44 (45), 7342-72.
9. Harding et al., Non-enzymic posttranslational modification of proteins in aging. A review. Mech Ageing Dev 1989, 50 (1), 7-16.
10. Pisupati et al., Biosimilarity under stress: A forced degradation study of Remicade® and Remsima™. mAbs 2017, 9 (7), 1197-1209.
11. Yang et al., Rapid assessment of oxidation via middle-down LCMS correlates with methionine side-chain solvent-accessible surface area for 121 clinical stage monoclonal antibodies. mAbs 2017, 9 (4), 646-653.
12. Sokolowska, I.; Mo, J.; Dong, J.; Lewis, M. J.; Hu, P., Subunit mass analysis for monitoring antibody oxidation. mAbs 2017, 9 (3), 498-505.
13. Yan et al. Isomerization and Oxidation in the Complementarity-Determining Regions of a Monoclonal Antibody: A Study of the Modification-Structure-Function Correlations by Hydrogen-Deuterium Exchange Mass Spectrometry. Anal Chem 2016, 88 (4), 2041-50.
14. Pavon et al., Selective Tryptophan Oxidation of Monoclonal Antibodies: Oxidative Stress and Modeling Prediction. Anal Chem 2019, 91 (3), 2192-2200.
15. Dyck et al., Forced Degradation Testing as Complementary Tool for Biosimilarity Assessment. Bioengineering (Basel) 2019, 6 (3).
16. Yang, H.; Zubarev, R. A., Mass spectrometric analysis of asparagine deamidation and aspartate isomerization in polypeptides. Electrophoresis 2010, 31 (11), 1764-72.
17. Riggs et al. Analysis of Glutamine Deamidation: Products, Pathways, and Kinetics. Anal Chem 2019, 91 (20), 13032-13038.
18. Li et al. Structural Elucidation of Post-Translational Modifications in Monoclonal Antibodies. In State-of-the-Art and Emerging Technologies for Therapeutic Monoclonal Antibody Characterization Volume 2, Schiel, J.; Davis, d.; Borisov, O., Eds. American Chemical Society: 2015.
19. Liu, D. T.-Y., Deamidation: a source of microheterogeneity in pharmaceutical proteins. Trends in Biotechnology 1992, 10, 364-369.
20. Shimizu, K.; Kita, A.; Fujii, N.; Miki, K., Structural features of isomerizable aspartyl residues in human alpha-crystallins. Mol Vis 2012, 18, 1823-7.
21. Fujii, N.; Sakaue, H.; Sasaki, H.; Fujii, N., A rapid, comprehensive liquid chromatography-mass spectrometry (LC-MS)-based survey of the Asp isomers in crystallins from human cataract lenses. J Biol Chem 2012, 287 (47), 39992-40002.
22. Masuda et al., D-Aspartic acid in bovine dentine non-collagenous phosphoprotein. Arch Oral Biol 2002, 47(11), 757-62.
23. Mishra, P. K. K.; Mahawar, M., PIMT-Mediated Protein Repair: Mechanism and Implications. Biochemistry (Mosc) 2019, 84 (5), 453-463.
24. Mishra et al. Isolation and Identification of Protein L-Isoaspartate- O-Methyltransferase (PIMT) Interacting Proteins in Salmonella Typhimurium. Curr Microbiol 2019.
25. Clarke, S., Aging as war between chemical and biochemical processes: protein methylation and the recognition of age-damaged proteins for repair. Ageing Res Rev 2003, 2 (3), 263-85.
26. Sakaue et al., Isomeric Replacement of a Single Aspartic Acid Induces a Marked Change in Protein Function: The Example of Ribonuclease A. ACS Omega 2017, 2 (1), 260-267.
27. Doyle, H. A.; Gee, R. J.; Mamula, M. J., Altered immunogenicity of isoaspartate containing proteins. Autoimmunity 2007, 40 (2), 131-7.
28. Roher et al., Structural alterations in the peptide backbone of betaamyloid core protein may account for its deposition and stability in Alzheimer's disease. J Biol Chem 1993, 268 (5), 3072-83.
29. Lambeth, T. R.; Riggs, D. L.; Talbert, L. E.; Tang, J.; Coburn, E.; Kang, A. S.; Noll, J.; Augello, C.; Ford, B. D.; Julian, R. R., Spontaneous Isomerization of Long-Lived Proteins Provides a Molecular Mechanism for the Lysosomal Failure Observed in Alzheimer's Disease. ACS Cent Sci 2019, 5 (8), 1387-1395.
30. Nowak, C.; J, K. C.; S, M. D.; Katiyar, A.; Bhat, R.; Sun, J.; Ponniah, G.; Neill, A.; Mason, B.; Beck, A.; Liu, H., Forced degradation of recombinant monoclonal antibodies: A practical guide. MAbs 2017, 9 (8), 1217-1230.
31. Hawe, A.; Wiggenhorn, M.; van de Weert, M.; Garbe, J. H.; Mahler, H. C.; Jiskoot, W., Forced degradation of therapeutic proteins. J Pharm Sci 2012, 101 (3), 895-913.
32. Carter, P. J., Introduction to current and future protein therapeutics: a protein engineering perspective. Exp Cell Res 2011, 317 (9), 1261-9.
33. Zhang, Z., Large-scale identification and quantification of covalent modifications in therapeutic proteins. Anal Chem 2009, 81 (20), 8354-64.
34. Lee, K. R.; Bongers, J.; Gulati, D.; Burman, S., Statistical validation of reproducibility of HPLC peptide mapping for the identity of an investigational drug compound based on principal component analysis. Drug Dev Ind Pharm 2000, 26 (10), 1045-57.
35. Bongers et al., Validation of a peptide mapping method for a therapeutic monoclonal antibody: what could we possibly learn about a method we have run 100 times? J Pharm Biomed Anal 2000, 21 (6), 1099-128.
36. Rehder et al., Isomerization of a single aspartyl residue of anti-epidermal growth factor receptor immunoglobulin gamma2 antibody highlights the role avidity plays in antibody activity. Biochemistry 2008, 47 (8), 2518-30.
37. Harris et al., Identification of multiple sources of charge heterogeneity in a recombinant antibody. J Chromatogr 8 Biomed Sci Appl 2001, 752 (2), 233-45.
38. Cacia, J.; Keck, R.; Presta, L. G.; Frenz, J., Isomerization of an aspartic acid residue in the complementarity-determining regions of a recombinant antibody to human IgE: identification and effect on binding affinity. Biochemistry 1996, 35 (6), 1897-903.
39. Robinson, C. J.; Jones, C., Quality control and analytical techniques for biopharmaceuticals. Bioanalysis 2011, 3 (1), 81-95.
40. Hui et al., Identification of Asp95 as the site of succinimide formation in recombinant human glial cell line-derived neurotrophic factor. Arch Biochem Biophys 1998, 358 (2), 377-84.
41. Lehmann et al., Analysis of isoaspartate in peptides by electrospray tandem mass spectrometry. Protein Sci 2000, 9 (11), 2260-8.
42. Chelius, D.; Rehder, D. S.; Bondarenko, P. V., Identification and characterization of deamidation sites in the conserved regions of human immunoglobulin gamma antibodies. Anal Chem 2005, 77 (18), 6004-11.
43. Yamazaki, Y.; Fujii, N.; Sadakane, Y.; Fujii, N., Differentiation and semiquantitative analysis of an isoaspartic acid in human alpha-Crystallin by postsource decay in a curved field reflectron. Anal Chem 2010, 82 (15), 6384-94.
44. DeGraan-Weber, N.; Zhang, J.; Reilly, J. P., Distinguishing Aspartic and Isoaspartic Acids in Peptides by Several Mass Spectrometric Fragmentation Methods. J Am Soc Mass Spectrom 2016, 27 (12), 2041-2053.
45. Gonzalez et al., Differentiating alpha- and beta-aspartic acids by electrospray ionization and low-energy tandem mass spectrometry. Rapid Commun Mass Spectrom 2000, 14 (22), 2092-102.
46. Eakin et al., Assessing analytical methods to monitor isoAsp formation in monoclonal antibodies. Front Pharmacol 2014, 5, 87.
47. Chan, W. Y.; Chan, T. W.; O'Connor, P. B., Electron transfer dissociation with supplemental activation to differentiate aspartic and isoaspartic residues in doubly charged peptide cations. J Am Soc Mass Spectrom 2010, 21 (6), 1012-5.
48. Grappin et al., New proteomic developments to analyze protein isomerization and their biological significance in plants. J Proteomics 2011, 74 (8), 1475-82.
49. Yu et al., In-Source Decay Characterization of Isoaspartate and beta-Peptides. Int J Mass Spectrom 2015, 390, 101-109.
50. Lambeth, T. R.; Julian, R. R., Differentiation of peptide isomers and epimers by radical-directed dissociation. Methods Enzymol 2019, 626, 67-87.
51. Riggs, D. L.; Gomez, S. V.; Julian, R. R., Sequence and Solution Effects on the Prevalence of d-Isomers Produced by Deamidation. ACS Chem Biol 2017, 12 (11), 2875-2882.
52. Zheng, X.; Deng, L.; Baker, E. S.; Ibrahim, Y. M.; Petyuk, V. A.; Smith, R. D., Distinguishing d- and laspartic and isoaspartic acids in amyloid beta peptides with ultrahigh resolution ion mobility spectrometry. Chem Commun (Camb) 2017, 53 (56), 7913-7916.
53. Ayrton et al., Gas Phase Ion Chemistry to Determine Isoaspartate in a Peptide Backbone. J Am Soc Mass Spectrom 2018, 29 (7), 1339-1344.
54. Pekov et al., Evaluation of MALDI-TOF/TOF Mass Spectrometry Approach for Quantitative Determination of Aspartate Residue Isomerization in the Amyloid-beta Peptide. J Am Soc Mass Spectrom 2019, 30 (7), 1325-1329.
55. Ivanov et al., Probabilistic model applied to ion abundances in product-ion spectra: quantitative analysis of aspartic acid isomerization in peptides. Anal Bioanal Chem 2019, 411 (29), 7783-7789.
56. Aichler, M.; Walch, A., MALDI Imaging mass spectrometry: current frontiers and perspectives in pathology research and practice. Lab Invest 2015, 95 (4), 422-31.
57. Ly et al., High-resolution MALDI mass spectrometric imaging of lipids in the mammalian retina. Histochem Cell Biol 2015, 143 (5), 453-62.
58. Sauget, M.; Valot, B.; Bertrand, X.; Hocquet, D., Can MALDI-TOF Mass Spectrometry Reasonably Type Bacteria? Trends Microbiol 2017, 25 (6), 447-455.
59. Heap et al., Identifying Inhibitors of Inflammation: A Novel High-Throughput MALDI-TOF Screening Assay for Salt-Inducible Kinases (SIKs). SLAS Discov 2017, 22 (10), 1193-1202.
60. Winter et al., Automated MALDI Target Preparation Concept: Providing Ultra-High-Throughput Mass Spectrometry- Based Screening for Drug Discovery. SLAS Technol 2019, 24 (2), 209-221.
61. Winter, et al., Establishing MALDI-TOF as Versatile Drug Discovery Readout to Dissect the PTP1B Enzymatic Reaction. SLAS Discov 2018, 23 (6), 561-573.
62. Suckau et al., A novel MALDI LIFT-TOF/TOF mass spectrometer for proteomics. Anal Bioanal Chem 2003, 376 (7), 952-65.
63. Wells, J. M.; McLuckey, S. A., Collision-induced dissociation (CID) of peptides and proteins. Methods Enzymol 2005, 402, 148-85.
64. Chaurand, P.; Luetzenkirchen, F.; Spengler, B., Peptide and protein identification by matrixassisted laser desorption ionization (MALDI) and MALDI-post-source decay time-of-flight mass spectrometry. J Am Soc Mass Spectrom 1999, 10 (2), 91-103.
65. Makarov, A., Electrostatic axially harmonic orbital trapping: a high-performance technique of mass analysis. Anal Chem 2000, 72 (6), 1156-62.
66. Hu et al., The Orbitrap: a new mass spectrometer. J Mass Spectrom 2005, 40 (4), 430-43.
67. Wysocki et al., Mobile and localized protons: a framework for understanding peptide dissociation. J Mass Spectrom 2000, 35 (12), 1399-406.
68. Indeykina et al., Capabilities of MS for analytical quantitative determination of the ratio of alpha- and betaAsp7 isoforms of the amyloid-beta peptide in binary mixtures. Anal Chem 2011, 83 (8), 3205-10.
69. Haberger et al., Assessment of chemical modifications of sites in the CDRs of recombinant antibodies: Susceptibility vs. functionality of critical quality attributes. MAbs 2014, 6 (2), 327-39.
70. Gahoual et al., Rapid and multi-level characterization of trastuzumab using sheathless capillary electrophoresistandem mass spectrometry. MAbs 2013, 5 (3), 479-90.
71. Papayannopoulos, I. A.; Biemann, K., Fast atom bombardment and tandem mass spectrometry of synthetic peptides and byproducts. Pept Res 1992, 5 (2), 83-90.
72. Hsu et al., Selective deamidation of recombinant human stem cell factor during in vitro aging: isolation and characterization of the aspartyl and isoaspartyl homodimers and heterodimers. Biochemistry 1998, 37 (8), 2251-62.
73. Smith, L. M., Is charge reduction in ESI really necessary? J Am Soc Mass Spectrom 2008, 19 (5), 629-31.
74. Rogers et al., Development of a quantitative mass spectrometry multi-attribute method for characterization, quality control testing and disposition of biologics. MAbs 2015, 7 (5), 881-90.

## Claims

1. A method of processing a protein, the method comprising:
(a) digesting a protein into peptides,
(b) subjecting the peptides to mass spectrometry to fragment a peptide bond N-terminal to an isoAsp and measure said fragmentation by the mass spectrometry;
(c) comparing the fragmentation to a threshold, wherein the fragmentation exceeding the threshold is indicative of the presence of the isoAsp in the protein; and
(d)
(i) rejecting the protein comprising the isoAsp wherein rejection comprises halting production of the protein or a purification process of the protein, or disposing of a supply of the protein, or
(ii) engineering the protein to remove the isoAsp.

2. The method of claim 1,
(i) further comprising charge reduction prior to (b),
and/or
(ii) wherein the peptides subject to mass spectrometry in (b) are singly-charged.

3. The method of claim 1 or 2, wherein the mass spectrometry is liquid chromatography-mass spectrometry/mass-spectrometry (LC-MS/MS),
optionally wherein the LC-MS/MS comprises electrospray ionization.

4. The method of any of the preceding claims,
(i) wherein the fragmentation is measured as a b-series peak, a y-series peak, or both;
(ii) wherein the mass spectrometry is matrix-assisted laser desorption/ionization time-of-flight/time-of-flight (MALDI-TOF/TOF);
(iii) wherein (a) comprises thermal degradation;
(iv) wherein (a) comprises enzymatic digestion with a proteolytic enzyme,
preferably wherein the proteolytic enzyme comprises at least one of chymotrypsin, trypsin, pepsin, papain, or elastase;
(v) wherein the isoAsp is not adjacent to an asparagine in the amino acid sequence of the protein and/or is not adjacent to a threonine in the amino acid sequence of the protein;
(vi) wherein the isoAsp is adjacent to an adjacent aspartate residue in the amino acid sequence of the protein,
preferably wherein the adjacent aspartate is isoAsp or L-Asp,
and/or
(vii) wherein the fragmentation exceeds the threshold when a b-peak and/or a y-peak of the fragmented peptide bond N-terminal to isoAsp is present.

5. The method of any of claims 1-4(vi), wherein the threshold is a fragmentation of a peptide bond N-terminal to an L-Asp in a control peptide,
optionally
(i) wherein the fragmentation of the peptide bond of the control peptide is provided as an electronically-stored value,
or
(ii) further comprising subjecting the control peptide to mass spectrometry, thereby obtaining the fragmentation of the peptide bond of the control peptide.

6. The method of claim 5, wherein the control peptide is a synthetic peptide.

7. The method of any of the preceding claims, wherein the protein is a large peptide, antibody, antibody fragment, antibody fusion peptide or antigen-binding fragment thereof.

8. The method of claim 7, wherein the antibody is a polyclonal or monoclonal antibody.

9. The method of any of the preceding claims,
(i) further comprising determining the position of the isoAsp in the protein;
(ii) wherein the isoAsp is detected in a protein discovery process or a protein production process;
(iii) wherein an absence of the isoAsp indicates suitability of the protein for further processing,
and/or
(iv) wherein the (d) rejecting the protein comprising the isoAsp comprises rejecting a quantity of product comprising the protein, or rejecting a clone that produces the protein.

10. The method of any of claims 1-9(iii), wherein (d) engineering the protein to remove the isoAsp comprises inducing a point mutation at the position of the isoAsp in the protein.

11. The method of any of the preceding claims,
(i) wherein the presence of isoAsp results in structural changes in the peptide backbone compared to a reference protein comprising an L-Asp at the same position;
(ii) wherein the presence of isoAsp inhibits potency of the protein;
(iii) wherein the presence of isoAsp increases the immunogenicity of the protein compared to a reference protein comprising an L-Asp at the same position;
(iv) wherein at least one of the peptides comprises a positive charge at the N terminus of the peptide,
and/or
(v) further comprising introducing a positive charge to the N terminus of the peptides.

12. The method of claim 11(v), wherein the positive charge is introduced by incubating the peptide with an N-terminal charge-derivatizing reagent.

13. The method of claim 12, wherein the N-terminal charge-derivatizing reagent is (N-succinimidyloxycarbonylmethyl) tris (2,4,6 trimethoxyphenyl) phosphonium bromide (TMPP) or a derivative thereof.

14. The method of claim 12, wherein the N-terminal charge-derivatizing reagent is tris[2,4,6-trimethoxyphenyl]phosphonium acetyl (TMPP-Ac) or a derivative thereof.

15. The method of any one of claims 11(iv)-14, wherein the *iso*Asp is within 5 amino acid residues of a C-terminus of at least one of the peptides.

## Patentansprüche

1. Verfahren zum Verarbeiten eines Proteins, wobei das Verfahren umfasst:
(a) Abbauen eines Proteins zu Peptiden,
(b) Unterziehen der Peptide einer Massenspektrometrie, um eine Peptidbindung N-terminal zu einem isoAsp zu fragmentieren und die Fragmentierung mittels der Massenspektrometrie zu messen,
(c) Vergleichen der Fragmentierung mit einem Schwellenwert, wobei die Fragmentierung, die den Schwellenwert überschreitet, auf das Vorhandensein des isoAsp im Protein hinweist, und
(d)
(i) Verwerfen des Proteins, welches das isoAsp umfasst, wobei das Verwerfen das Beenden der Produktion des Proteins oder eines Reinigungsprozesses des Proteins oder das Entsorgen eines Vorrats des Proteins umfasst, oder
(ii) Modifizieren des Proteins, um das isoAsp zu entfernen.

2. Verfahren nach Anspruch 1,
(i) welches ferner eine Ladungsreduktion vor (b) umfasst,
und/oder
(ii) wobei die Peptide, die in (b) der Massenspektrometrie unterzogen werden, einfach geladen sind.

3. Verfahren nach Anspruch 1 oder 2, wobei die Massenspektrometrie eine Flüssigchromatographie-Massenspektrometrie/Massenspektrometrie (LC-MS/MS) ist, wobei die LC-MS/MS wahlweise eine Elektrospray-Ionisierung umfasst.

4. Verfahren nach einem der vorstehenden Ansprüche,
(i) wobei die Fragmentierung als Peak der ab-Serie, als Peak der y-Serie oder als beides gemessen wird,
(ii) wobei die Massenspektrometrie eine matrixunterstützte Laserdesorptions-/Ionisations-Flugzeit-/Flugzeit-Massenspektrometrie (MALDI-TOF/TOF) ist,
(iii) wobei (a) eine thermische Zersetzung umfasst,
(iv) wobei (a) einen enzymatischen Abbau mit einem proteolytischen Enzym umfasst,
vorzugsweise wobei das proteolytische Enzym mindestens eines von Chymotrypsin, Trypsin, Pepsin, Papain oder Elastase umfasst,
(v) wobei das isoAsp nicht benachbart zu einem Asparagin in der Aminosäuresequenz des Proteins und/oder nicht benachbart zu einem Threonin in der Aminosäuresequenz des Proteins ist,
(vi) wobei das isoAsp benachbart zu einem benachbarten Aspartatrest in der Aminosäuresequenz des Proteins ist,
vorzugsweise wobei das benachbarte Aspartat isoAsp oder L-Asp ist und/oder
(vii) wobei die Fragmentierung den Schwellenwert überschreitet, wenn ein b-Peak und/oder ein y-Peak der fragmentierten Peptidbindung N-terminal zu isoAsp vorhanden ist.

5. Verfahren nach einem der Ansprüche 1 bis 4(vi), wobei der Schwellenwert eine Fragmentierung einer Peptidbindung N-terminal zu einem L-Asp in einem Kontrollpeptid ist,
wahlweise:
(i) wobei die Fragmentierung der Peptidbindung des Kontrollpeptids als elektronisch gespeicherter Wert bereitgestellt wird,
oder
(ii) ferner umfassend das Unterziehen des Kontrollpeptids einer Massenspektrometrie, wodurch die Fragmentierung der Peptidbindung des Kontrollpeptids erhalten wird.

6. Verfahren nach Anspruch 5, wobei das Kontrollpeptid ein synthetisches Peptid ist.

7. Verfahren nach einem der vorstehenden Ansprüche, wobei das Protein ein großes Peptid, ein Antikörper, ein Antikörperfragment, ein Antikörperfusionspeptid oder ein Antigen-bindendes Fragment davon ist.

8. Verfahren nach Anspruch 7, wobei der Antikörper ein polyklonaler oder monoklonaler Antikörper ist.

9. Verfahren nach einem der vorstehenden Ansprüche,
(i) ferner umfassend das Bestimmen der Position des isoAsp in dem Protein,
(ii) wobei das isoAsp in einem Protein-Entdeckungsprozess oder einem Protein-Produktionsprozess nachgewiesen wird,
(iii) wobei das Fehlen des isoAsp die Eignung des Proteins für die weitere Verarbeitung anzeigt,
und/oder
(iv) wobei das (d) Verwerfen des Proteins, welches das isoAsp umfasst, das Verwerfen einer Menge des Produkts, welches das Protein umfasst, oder das Verwerfen eines Klons, der das Protein produziert, umfasst.

10. Verfahren nach einem der Ansprüche 1 bis 9(iii), wobei (d) das Modifizieren des Proteins, um das isoAsp zu entfernen, das Induzieren einer Punktmutation an der Position des isoAsp in dem Protein umfasst.

11. Verfahren nach einem der vorstehenden Ansprüche,
(i) wobei das Vorhandensein von isoAsp zu strukturellen Veränderungen im Peptidgerüst im Vergleich zu einem Referenzprotein führt, welches an derselben Position ein L-Asp umfasst,
(ii) wobei das Vorhandensein von isoAsp die Wirksamkeit des Proteins hemmt,
(iii) wobei das Vorhandensein von isoAsp die Immunogenität des Proteins im Vergleich zu einem Referenzprotein erhöht, welches an derselben Position ein L-Asp umfasst,
(iv) wobei mindestens eines der Peptide eine positive Ladung am N-Terminus des Peptids umfasst,
und/oder
(v) ferner umfassend das Einführen einer positiven Ladung in den N-Terminus der Peptide.

12. Verfahren nach Anspruch 11(v), wobei die positive Ladung durch Inkubieren des Peptids mit einem N-terminal ladungsderivatisierenden Reagenz eingeführt wird.

13. Verfahren nach Anspruch 12, wobei das N-terminal ladungsderivatisierende Reagenz (N-Succinimidyloxycarbonylmethyl)tris(2,4,6-trimethoxyphenyl)phosphoniumbromid (TMPP) oder ein Derivat davon ist.

14. Verfahren nach Anspruch 12, wobei das N-terminal ladungsderivatisierende Reagenz Tris[2,4,6-trimethoxyphenyl]phosphoniumacetyl (TMPP-Ac) oder ein Derivat davon ist.

15. Verfahren nach einem der Ansprüche 11(iv) bis 14, wobei sich das Asp innerhalb von 5 Aminosäureresten eines C-Terminus von mindestens einem der Peptide befindet.

## Revendications

1. Procédé de traitement d'une protéine, le procédé comprenant :
(a) une digestion d'une protéine en peptides,
(b) une soumission des peptides à une spectrométrie de masse pour fragmenter une extrémité N-terminale de liaison peptidique en *isoAsp* (isoaspartate) et mesurer ladite fragmentation par la spectrométrie de masse ;
(c) une comparaison de la fragmentation à un seuil, dans lequel la fragmentation dépassant le seuil est indicative de la présence de l'*iso*Asp dans la protéine ; et
(d)
(i) un rejet de la protéine comprenant l'*iso*Asp dans lequel un rejet comprend un arrêt de production de la protéine ou un processus de purification de la protéine, ou une mise au rebut d'une fourniture de la protéine, ou
(ii) une ingénierie de la protéine pour retirer l'*iso*Asp.

2. Le procédé de la revendication 1,
(i) comprenant en outre une réduction de charge avant (b) ,
et/ou
(ii) dans lequel les peptides soumises à une spectrométrie de masse en (b) sont chargées individuellement.

3. Le procédé de la revendication 1 ou 2, dans lequel la spectrométrie de masse est une chromatographie liquide - spectrométrie de masse/spectrométrie de masse (LC-MS / MS) ,
facultativement dans lequel la LC-MS/MS comprend une ionisation par électronébulisation.

4. Le procédé de l'une quelconque des revendications précédentes,
(i) dans lequel la fragmentation est mesurée comme un pic de série b, un pic de série y, ou les deux ;
(ii) dans lequel la spectrométrie de masse est une spectrométrie de masse à temps de vol pour la désorption/ionisation laser assistée par matrice/à temps de vol (MALDI-TOF/TOF) ;
(iii) dans lequel (a) comprend une dégradation thermique ;
(iv) dans lequel (a) comprend une digestion enzymatique avec un enzyme protéolytique,
de préférence dans lequel l'enzyme protéolytique comprend au moins un de chymotrypsine, trypsine, pepsine, papaïne ou élastase ;
(v) dans lequel l'*iso*Asp n'est pas adjacent à une asparagine dans la séquence d'acide aminé de la protéine et/ou n'est pas adjacent à une thréonine dans la séquence d'acide aminé de la protéine ;
(vi) dans lequel l'*iso*Asp est adjacent à un résidu d'aspartate adjacent dans la séquence d'acide aminé de la protéine,
de préférence dans lequel l'aspartate adjacent est un *isoAsp* ou un L-Asp,
et/ou
(vii) dans lequel la fragmentation dépasse le seuil où un pic b et/ou un pic y de l'extrémité N-terminale de liaison peptidique fragmentée en *isoAsp* est présent.

5. Le procédé de l'une quelconque des revendications 1 à 4 (vi), dans lequel le seuil est une fragmentation d'une extrémité N-terminale de liaison peptidique en L-Asp dans un peptide de contrôle,
facultativement
(i) dans lequel la fragmentation de la liaison peptidique du peptide de contrôle est fournie en tant que valeur stockée électroniquement,
ou
(ii) comprenant en outre une soumission du peptide de contrôle à une spectrométrie de masse, obtenant ainsi la fragmentation de la liaison peptidique du peptide de contrôle.

6. Le procédé de la revendication 5, dans lequel le peptide de contrôle est un peptide synthétique.

7. Le procédé de l'une quelconque des revendications précédentes, dans lequel la protéine est un grand peptide, un anticorps, un fragment d'anticorps, un peptide de fusion d'anticorps, ou un fragment de liaison d'antigène de celle-ci.

8. Le procédé de la revendication 7, dans lequel l'anticorps est un anticorps polyclonal ou monoclonal.

9. Le procédé de l'une quelconque des revendications précédentes,
(i) comprenant en outre une détermination de la position de l'*iso*Asp dans la protéine ;
(ii) dans lequel l'*iso*Asp est détecté dans un processus d'exploration de protéine ou un processus de production de protéine ;
(iii) dans lequel une absence de l'*iso*Asp indique une aptitude de la protéine à un traitement plus avant,
et/ou
(iv) dans lequel le (d) rejet de la protéine comprenant l'*iso*Asp comprend un rejet d'une quantité de produit comprenant la protéine, ou un rejet d'un clone qui produit la protéine.

10. Le procédé de l'une quelconque des revendications 1 à 9 (iii), dans lequel (d) une ingénierie de la protéine pour retirer l'*iso*Asp comprend une induction d'une mutation ponctuelle sur la position de l'*iso*Asp dans la protéine.

11. Le procédé de l'une quelconque des revendications précédentes,
(i) dans lequel la présence d'*iso*Asp entraîne des modifications structurelles dans le squelette peptidique en comparaison d'une protéine de référence comprenant un L-Asp sur la même position ;
(ii) dans lequel la présence de l'*iso*Asp inhibe une puissance de la protéine ;
(iii) dans lequel la présence d'*iso*Asp augmente l'immunogénicité de la protéine en comparaison d'une protéine de référence comprenant un L-Asp sur la même position ;
(iv) dans lequel au moins un des peptides comprend une charge positive au niveau de l'extrémité N terminale du peptide,
et/ou
(v) comprenant en outre une introduction d'une charge positive à l'extrémité N terminale des peptides.

12. Le procédé de la revendication 11 (v), dans lequel la charge positive est introduite en incubant le peptide avec un réactif de dérivatisation de charge d'extrémité N terminale.

13. Le procédé de la revendication 12, dans lequel le réactif de dérivatisation de charge d'extrémité N terminale est un bromure de phosphonium (N-succinimidyloxycarbonylméthyle) tris (2,4,6 triméthoxyphényle) (TMPP) ou un dérivé de celui-ci.

14. Le procédé de la revendication 12, dans lequel le réactif de dérivatisation de charge d'extrémité N terminale est un acétyle de phosphonium tris[2,4,6-triméthoxyphényle] (TMP PP-Ac) ou un dérivé de celui-ci.

15. Le procédé de l'une quelconque des revendications 11 (iv) à 14, dans lequel l'isoAsp est présent dans 5 résidus d'acide aminé d'une extrémité C terminale d'au moins un des peptides.
